# EUROPÄISCHE PATENTSCHRIFT

(11) **EP 1 297 165 B1**
(45) Veröffentlichungstag und Bekanntmachung des Hinweises auf die Patenterteilung: **01.03.2006**
(21) Anmeldenummer: 01969333.2
(22) Anmeldetag: 03.07.2001
(51) Int. Cl.: C12N 15/82, C12N 5/10, A01H 5/00

(54) **PROMOTOREN ZUR GENEXPRESSION IN KARYOPSEN VON PFLANZEN**
PROMOTERS FOR GENE EXPRESSION IN CARYOPSES OF PLANTS
PROMOTEURS D'EXPRESSION GENIQUE DANS LES CARYOPSES DES PLANTES

(30) Priorität: 06.07.2000 DE 10032379
(43) Veröffentlichungstag der Anmeldung: 02.04.2003
(73) Patentinhaber: Bayer CropScience GmbH, 65926 Frankfurt (DE)
(72) Erfinder: SPRUNCK, Stephanie, 22607 Hamburg (DE); KLUTH, Antje, 10407 Berlin (DE); BECKER, Dirk, 21149 Hamburg (DE); LUETTICKE, Stephanie, 20099 Hamburg (DE); LOERZ, Horst, 22589 Hamburg (DE)
(86) Internationale Anmeldenummer: PCT/EP2001/007593
(87) Internationale Veröffentlichungsnummer: WO 2002/002786

(56) Entgegenhaltungen:
- WO-A-00/66745
- WO-A-97/45545
- WO-A-99/14314
- US-A- 6 013 862
- LI Z ET AL: "Cloning and characterization of a gene encoding wheat starch synthase I." THEORETICAL AND APPLIED GENETICS, Bd. 98, Nr. 8, Juni 1999 (1999-06), Seiten 1208-1216, XP002184090 ISSN: 0040-5752 in der Anmeldung erwähnt
- DATABASE EMBL SEQUENCE DATABASE [Online] 18. Oktober 1996 (1996-10-18) WALTER L., LOERZ H., LUETTICKE S.T.: "Triticum aestivum starch synthase mRNA, partial cds. - "Sequence analysis of a cDNA coding for a starch synthase of wheat" " XP002184092 in der Anmeldung erwähnt
- LI ZHONGYI ET AL: "The localization and expression of the class II starch synthases of wheat." PLANT PHYSIOLOGY (ROCKVILLE), Bd. 120, Nr. 4, August 1999 (1999-08), Seiten 1147-1155, XP002184091 ISSN: 0032-0889 in der Anmeldung erwähnt
- DENYER K ET AL: "IDENTIFICATION OF MULTIPLE ISOFORMS OF SOLUBLE AND GRANULE-BOUND STARCH SYNTHASE IN DEVELOPING WHEAT ENDOSPERM" PLANTA, SPRINGER VERLAG, DE, Bd. 196, 1995, Seiten 256-265, XP002043126 ISSN: 0032-0935
- DATABASE EMBL SEQUENCE DATABASE [Online] 30. Juli 2000 (2000-07-30) ANDERSON, O.A., ET AL. : "International Triticeae EST Cooperative (ITEC): Production of Expressed Sequence Tags for Species of the Triticeae" XP002184093

## Beschreibung

Die vorliegende Erfindung betrifft Promotoren, die eine karyopsenspezifische Expression oder Suppression von Genen in genetisch modifizierten Pflanzen erlauben, Verfahren zur gewebespezifischen Genexpression oder Gensuppression in Pflanzen, Expressionskassetten, rekombinante Vektoren und Wirtszellen, die solche Promotoren enthalten, mit besagten Promotoren transformierte transgene Pflanzenzellen und Pflanzen, sowie Verfahren zur Herstellung solcher Pflanzenzellen und Pflanzen.

Nachfolgend werden Dokumente aus dem Stand der Technik zitiert, deren Offenbarungsgehalt hiermit durch Referenz Teil dieser Anmeldung ist.

Der Einsatz von Pflanzen, die mit Hilfe gentechnischer Verfahren in ihrem Erbgut verändert wurden, hat sich in vielen Bereichen der Landwirtschaft als vorteilhaft erwiesen, um bestimmte Eigenschaften auf Nutzpflanzen zu übertragen. Die vomehmlichen Ziele sind zum einen der Pflanzenschutz und zum anderen eine Qualitäts- und Ertragssteigerung der Pflanzen bzw. der Produkte, die geerntet werden können.

Zahlreiche Verfahren zur genetischen Modifikation dikotyler und monokotyler Pflanzen sind bekannt (vgl. u.a. Gasser und Fraley, Science 244 (1989), 1293-1299; Potrykus, Ann. Rev. Plant Mol. Biol. Plant Physiol. 42 (1991), 205-225, Newell, Mol. Biotechnol. (2000),16(1) 53-65).

Oft basieren diese auf der Übertragung von Genkonstrukten, die in den meisten Fällen Kombinationen von bestimmten kodierenden Regionen von Strukturgenen mit Promotorregionen sowie Transkriptionsterminatoren derselben oder anderer (z.B. heterologer) Strukturgene darstellen.

Die Bereitstellung von Promotoren ist im Zusammenhang mit der Expression von Strukturgenen von großer Bedeutung für die Herstellung von transgenen Pflanzen, da die Spezifität eines Promotors ausschlaggebend dafür ist, zu welchem Zeitpunkt, in welchen Gewebetypen, unter welchen physiologischen Bedingungen und mit welcher Intensität ein transferiertes Gen in einer modifizierten Pflanze exprimiert wird.

Zur Bewältigung dieser unterschiedlichen Ansätze zur genetischen Veränderung von Pflanzen ist es daher erforderlich, Gene, die unterschiedlich reguliert werden sollen, unter die Kontrolle entsprechender Promotoren zu stellen.

Die Initiation und Regulation der Transkription unterliegt dem als Promotor bezeichneten DNA-Abschnitt eines Gens. In der Regel liegen Promotorsequenzen im 5'-flankierenden Bereich eines transkribierten Gens. Einzelne Elemente eines Promotors (z.B. transkriptionelle Enhancer) können auch im 3'-flankierenden Bereich oder innerhalb von Intron-Sequenzen eines Gens lokalisiert sein (Kuhlemeier (1992) Plant Mol. Biol. 19: 1-14; Luehrsen (1994) The Maize Handbook, 636-638).

Die kontrollierte Expression von Transgenen ist zum Beispiel für das Einführen von Resistenzeigenschaften oder die Modifikation von Stoffwechselvorgängen in Pflanzen von großem Nutzen. Soll ein Transgen oder dessen Genprodukt in definierte Stoffinrechselwege einer Pflanze eingreifen, z.B. einen neuen Inhaltstoff produzieren oder vor Pathogenbefall schützen, ist seine räumlich und/oder zeitlich kontrollierte Expression nur unter Verwendung eines induzierbaren und/oder gewebe- und/oder entwicklungsspezifischen Promotors möglich. Erst dadurch wird die gezielte Produktion von erwünschten Inhaltsstoffen in einem definierten Entwicklungsstadium oder innerhalb eines bestimmten Gewebes der Pflanze ermöglicht. Z. B. kann für die Anwendung der Antisense-Technologie, in der die Expression von pflanzeneigenen Genen verhindert werden soll, der Einsatz von gewebe- und/oder entwicklungsspezifischen Promotoren gegenüber einer gewebe- und/oder entwicklungsunabhängigen Expression von Vorteil, wenn z.B. der Antisense-Effekt genau in dem Entwicklungsstadium bzw. Gewebe der Pflanze auftritt, in dem auch das pflanzeneigene Gen exprimiert wird.

Eine Vielzahl von Promotoren, die die Expression von transferierten Genen oder Strukturgenen in Pflanzen steuern können, ist bereits bekannt. Der am häufigsten verwendete Promotor ist der 35S CaMV-Promotor (Franck et al., Cell 1 (1980), 285-294), der zu einer konstitutiven Expression des eingeführten Gens führt.
Häufig werden auch induzierbare Promotoren eingesetzt, beispielsweise zur Wundinduktion (DE-A-3843628), chemischen Induktion (Ward et al., Plant Molec. Biol. 22 (1993), 361-366) oder Lichtinduktion (Fluhr et al., Science 232 (1986), 1106-1112).

Die Verwendung der oft beschriebenen konstitutiven Promotoren (z.B. 35 S) ist unter bestimmten Umständen mit gewissen Nachteilen verbunden. Promotoren, die eine konstitutive Expression der von ihnen kontrollierten Gene bewirken, können beispielsweise zur Erzeugung herbizidtoleranter und pathogenresistenter Pflanzen eingesetzt werden, haben aber den Nachteil, daß die Produkte der von ihnen kontrollierten Gene in allen Teilen der Pflanze vorliegen, was unerwünscht sein kann, z.B. wenn die Pflanzen der Ernährung dienen sollen. Ein negativer Aspekt der gewebe- und/oder entwicklungsunabhängigen Expression eines Transgens kann außerdem in einer unerwünschten Wirkung auf die Pflanzenentwicklung bestehen. Induzierbare Promotoren sind in der Anwendung gleichfalls mit Nachteilen verbunden, da die Induktionsbedingungen bei landwirtschaftlich genutzten Pflanzen im Freiland typischerweise schwer kontrollierbar sind.

Auch die Verwendung zell- und gewebespezifischer Promotoren wurde beschrieben: schließzellenspezifische (DE-A-4207358), samen-, knollen- und fruchtspezifische (zusammengefaßt in Edwards and Coruzzi, Annu. Rev. Genet. 24 (1990), 275-303; DE-A-3843627), phloemspezifische (Schmülling et al., Plant Cell 1 (1989), 665-670), wurzelknöllchenspezifische (DE-A-3702497) oder meristemspezifische Promotoren (Ito et al., Plant Mol. Biol. 24 (1994),863-878).

Promotoren, die die Genexpression in der Karyopse regulieren, sind bisher in begrenzter Zahl bekannt. Zur Bewältigung bestimmter Ansätze der genetischen Veränderung von Pflanzen ist es daher erforderlich, alternative Promotorsysteme zur Genexpression in der Karyopse zur Verfügung zu stellen, die im Vergleich zu den bekannten Promotoren unterschiedlich reguliert sind.

Aus verschiedenen Pflanzenspezies wurden bereits Gene der Stärkebiosynthese isoliert, deren Genprodukte spezifisch im Speichergewebe der Karyopse, nicht aber in vegetativen Geweben exprimiert werden, z.B. entsprechende Gene bzw. cDNA-Klone der GBSS I. Dazu gehört der *waxy* Locus aus Mais (Klösgen *et al*. (1986) Mol. Gen. Genet. 203: 237-244), sowie aus Gerste (Rohde *et al.* (1988) Nucleic Acid Research 16, No. 14: 7185-7186), Reis (Wang *et al.* (1990) Nucleic Acid Research 18: 5898), Kartoffel (van der Leij *et al.* (1991) Mol. Gen. Genet. 228: 240-248), Erbse (Dry *et al.* (1992) Plant J. 2: 193-202), Maniok (Salehuzzaman *et al*. (1993) Plant Mol. Biol. 20: 947-962), Hirse (Hsingh *et al*. (1995) EMBL Datenbank Acc.No. U23954) und Zuckerrübe (Schneider *et al.* (1999) Mol. Gen. Genet. 262: 515-524). Die Klonierungund Charakterisierung eines Gens der Stärkesynthase I aus Weizen wurde von Li et al. (1999, Theor. Appl. Genet. 98: 1208-1216) beschrieben.

Zu den Genprodukten, die spezifisch in der Karyopse expriomiert werden gehört auch die Starkesynthase vom Typ II (ssll). Entsprechende Gene wurden aus Mais (zSSIIa und zSSIIb; Harn *et al.* (1998) Plant Mol. Biol. 37: 639-649), Erbse (Dry *et al.* (1992) Plant J. 2: 193-202), Kartoffel (Edwards *et al.* (1995) Plant J. 8: 283-294) und Süßkartoffel (Ham *et al.* (1998) Acc. Nr. AF068834) isoliert.

Für Weizen stellt sich folgende Situation dar: Es wurden sowohlCDNA Klone für das *waxy*-Gen als auch für das ssll Gen isoliert und sequenziert. Insgesamt wurden 3 verschiedene cDNA klone ür die gbss1 aus Weizen isoliert (Clark *et al*. (1991) Plant Mol. Biol. 16: 1099-1101; Ainsworth *et al*. (1993) Plant Mol. Biol. 22: 67-82 (Block (1997) "Isolierung, Charakterisierung und Expressionsanalysen von Stärkesynthase-Genen aus Weizen" *(Triticum aestivum* L.), Dissertation, Universität Hamburg).

Darüber hinaus sind auch kodierende Sequenzen einer Stärkesynthase des Typ II (ssll) aus einer karyopsenspezifischen cDNA-Bank isoliert worden und es wurde deren karyopsenspezifische Expression nachgewiesen. In Northern-Analysen wurde gezeigt, daß die Transkripte der GBSS I (Block (1997) Dissertation, Universität Hamburg, Fachbereich Biologie) und der ssll (Walter (2000), Dissertation Uni Hamburg, Fachbereich Biologie, WO 97/45545, EMBL Datenbank Acc. No. U66377) in frühen Entwicklungsstadien der Karyopse, aber nicht im assimilierenden Blattgewebe auftreten. Für die ssll konnten darüber hinaus Transkripte im Endosperm und Perikarp gezeigt werden.

Drei cDNA-Sequenzen der ssll aus Weizen *(T. aestivum* L. cv. "Wyuna"; wSSll-A1, wSSll-B1, wSSll-D1) wurden ferner aus einer endospermspezifischen cDNA-Bank isoliert (Li *et al*., (1999) Plant Phys. 120: 1147-1155). Mittels PCR-Analysen wurde jeder der drei Klone einem Genom des hexaploiden Weizens zugeordnet. In Westem-Blot Analysen konnte gezeigt werden, daß das 100kDa Protein (SGP-B1) in frühen Stadien der Endospermentwicklung sowohl stärkekomgebunden, als auch in löslichen Form vorliegt. Inzwischen wurde die Isolierung und Charakterisierung von weiteren ssll-cDNA-Klonen (Triticum aestivum L. cv. "Fielder", Ss2a-1, Ss2a-2, Ss2a-3) beschrieben (Gao & Chibbar (2000) Genome 43: 768-775.

Ein cDNA-Klon einer stärkekorngebundenen Stärkesynthase vom Typ II (GBSS II), die nicht im Endosperm, sondern nur in Blättern und im Perikarp von Weizen exprimiert wird, konnte kürzlich isoliert werden (Vrinten & Nakamura (2000) Plant Physiol.122: 255-263). Spezifischeregulatorische Elemente des Aleurons von Weizen konnten aus dem Lipid transfer protein (Ltp) W1-Gen gewonnen werden (US 6,013,862). In diploidem Weizen (*Triticum monococcum* L.) hatten Denyer et al (1995, Planta 196: 256-265) mehrere Isoformen der Stärkesynthase (GBSS und SS) identifiziert. Es wurde außerdem auf Proteinebene eine 56kDa große Isoform einer GBSS beschrieben (Fujita & Taira (1998, Planta 207: 125-132). Diese isoform kann im Perikarp, Aleuron und Embryo von unreifen Karyopsen nachgewiesen werden.

Weiterhin bekannt sind Strukturen und Sequenzen von Ver- und Entzweigungsenzymen (WO 99/14314) sowie EST-Sequenzen von Durum-Weizen (Anderson et al (2000), NCBI Datenbank Acc.No. BE428407).

Während inzwischen drei homologe *waxy* Strukturgene, die auf den Chromosomen 7A, 4A und 7D des hexaploiden Weizes liegen, isoliert wurden (Murai *et al.* (1999) Gene 234: 71-79), sind die Promotorsequenzen dieser oder anderer genomischer Klone aus Weizen bisher unbekannt. Bekannt sind lediglich die 5'-flankierenden Bereiche der GBSS I aus Gerste (GenBank Acc.No. X07931), Löwenmäulchen (GenBank Acc.No. AJ006294), Reis (GenBank Acc.No. AB008794, AB008795), Kartoffel (GenBank Acc.No. X58453) und Mais (GenBank Acc.No. X03935).

WO 00/66745 offenbart, dass die mRNA-Expression der Weizen SSII mit Abstand am höchsten in Blättern ist.

Zur Lösung komplexer Aufgabestellungen im Zusammenhang mit der Expression von Genen in genetisch modifizierten Organismen ist es daher erforderlich, aus verschiedenen-Promotorsystemen unterschiedlicher Spezifität auswählen zu können. Hierzu leistet die vorliegende Erfindung einen Beitrag.

Der vorliegenden Erfindung liegt somit die Aufgabe zugrunde, Mittel bereitzustellen, die eine gezielte karyopsenspezifische Genexpression in genetisch modifizierten Pflanzen ermöglichen, vorzugsweise in monokotylen Pflanzen.

Durch den Einsatz der erfindungsgemäßen Mittel, d.h. der erfindungsgemäßen Nukleinsäuremoleküle, Vektoren, Zellen oder Pflanzen, wird ein gewebe- und/oder entwicklungsspezifisch definierter Eingriff in den pflanzlichen Stoffwechsel ermöglicht, z.B. in die Biosynthese von Speicherstärke, -fetten oder-proteinen oder auch die Nutzung der Karyopse als Speicher- oder Syntheseorgan für Reservestoffe (z.B. Polyglukane, Stärke, Fettsäuren, Fette, ggf. modifizierte Speicherproteine oder biopolymere Kunststoffe) ermöglicht.

Unter der Kontrolle der erfindungsgemäßen Nukleinsäuremoleküle bzw. Promotorsequenzen können somit Gene, insbesondere während der Komentwicklung von Getreiden, spezifisch und zu einem frühen Zeitpunkt in der Karyopse exprimiert werden.

Mittels der erfindungsgemäßen Promotorsequenzen können darüber hinaus Gene, insbesondere während der Komentwicklung von Getreiden, spezifisch und zu einem frühen Zeitpunkt in der Karyopse durch sog. gene silencing'-Strategien (Cosuppression) supprimiert werden. Cosuppressionsstrategien unter Einsatz von Promotoren sind ausführlich von Vaucheret et al. (Vaucheret et al., 1998, 16(6), 651-659) beschrieben worden. Insbesondere der Abschnitt, Transcriptional trans inactivation auf Seite 652 des Artikels von Vaucheret et al. sei hiermit durch Referenz Teil dieser Anmeldung, der speziell Cosuppressionsstrategien beschreibt, für die die erfindungsgemäßen Promotoren geeignet sind, insbesondere solche, die dort als ,ectopic trans-inactivation' bezeichnet werden können (Matzke et al., 1994, Mol. Gen. Genet. 244, 219-229). Solchermaßen können die erfindungsgemäßen Promotoren dazu verwendet werden, die Genexpression beliebiger Gene zu supprimieren, die unter der Kontrolle eines Promotors stehen, der als Ziel für die Cosuppression durch die erfindungsgemäßen Promotoren zugänglich ist. Gegebenenfalls ist hierfür bereits ein Sequenzabschnitt von nur etwa 90 bp Länge ausreichend.

Die erfindungsgemäßen Promotoren ermöglichen somit gezielte Veränderungen der Speicherstärke. Um darüber hinaus eine möglichst vielfältige Anwendung von Stärke für unterschiedliche industriellen Bedürfnisse zu ermöglichen, ist es wünschenswert Pflanzen bereitzustellen, die Stärken mit definierten Eigenschaften synthetisieren. So werden z.B. für die verarbeitende Industrie entscheidende Eigenschaften wie Löslichkeit, Verkleisterungsverhalten, Retrogradierungstendenz, Viskosität und Komplexiervermögen durch das Verhältnis von Amylose und Amylopektin zueinander, dem Verzweigungsgrad des Amylopektins und die Derivatisierung der Polymere bestimmt. Eine gezielte Modifizierung solcher Eigenschaften ersetzt aufwendige Verfahren zur Trennung von Amylose und Amylopektin oder die kostspielige chemische Modifizierung von Stärke.

Eine begrenzte Möglichkeit, Pflanzen mit veränderter Speicherstärke zu erhalten, besteht in der Anwendung klassischer Züchtungsmethoden. Durch Kreuzung spontan auftretender Mutanten gelang zum Beispiel die Herstellung eines (amylosefreien) "*waxy*" Weizens (Nakamura *et al*. (1995) Mol. Gen. Genet. 248: 253-259). Aufgrund des polyploiden Charakters des kommerziell bedeutenden Brot-Weizens sind Mutationen, welche die Stärkestruktur betreffen, nicht leicht zu erkennen, da sie von intakten Allelen kompensiert werden. Die Anwendung klassischer Züchtungsmethoden erweist sich daher als schwierig. Außerdem kann nur auf bereits vorhandene Enzymaktivitäten zurückgegriffen werden. Neue Aktivitäten, die bisher nicht in Pflanzen identifiziert wurden oder die in Pflanzen (oder anderen Organismen) identifiziert wurden, die nicht mit der Zielpflanze kreuzbar sind, können ebenfalls nicht mit Hilfe züchterischer Verfahren bearbeitet werden.

Eine Alternative zu den klassischen Züchtungsmethoden besteht in der gezielten Modifikation stärkeproduzierender Pflanzen durch gentechnische Verfahren. Voraussetzung hierfür ist jedoch neben der Identifizierung und Isolierung von Genen, deren Genprodukte an der Stärkesynthese und/oder Stärkemodifikation beteiligt sind, der Einsatz spezifischer Promotoren, die eine gewebe- und/oder entwicklungsspezifische Expression der von ihnen kontrollierten Gene in den stärkebildenden Geweben vermitteln.

Außerdem können durch den Einsatz der erfindungsgemäßen Promotorsequenzen Gene in das Pflanzengenom integriert werden, die dem Getreideendosperm eine modifizierte Funktion als Speichergewebe verleihen, z.B. zur Speicherung von Speicherstoffen, die nicht zu den Stärken zugeordnet werden können.

Diese Aufgaben werden erfindungsgemäß durch die in den Patentansprüchen charakterisierten Ausführungsformen gelöst.

Es wurde im Rahmen der vorliegenden Erfindung gefunden, daß überraschenderweise ein Promotor wie nachfolgend definiert eine karyopsenspezifische Expression einer von ihm kontrollierten kodierenden Nucleotidsequenz in Pflanzen bewirkt.

Eine Alternative zu den klassischen Züchtungsmethoden besteht in der gezielten Modifikation stärkeproduzierender Pflanzen durch gentechnische Verfahren. Voraussetzung hierfür ist jedoch neben der Identifizierung und Isolierung von Genen, deren Genprodukte an der Stärkesynthese und/oder Stärkemodifikation beteiligt sind, der Einsatz spezifischer Promotoren, die eine gewebe- und/oder entwicklungsspezifische Expression der von ihnen kontrollierten Gene in den stärkebildenden Geweben vermitteln.

Außerdem können durch den Einsatz der erfindungsgemäßen Promotorsequenzen Gene in das Pflanzengenom integriert werden, die dem Getreideendosperm eine modifizierte Funktion als Speichergewebe verleihen, z.B. zur Speicherung von Speicherstoffen, die nicht zu den Stärken zugeordnet werden können.

Diese Aufgaben werden erfindungsgemäß durch die in den Patentansprüchen charakterisierten Ausführungsformen gelöst.

Es wurde im Rahmen der vorliegenden Erfindung gefunden, daß überraschenderweise ein Promotor wie nachfolgend definiert eine karyopsenspezifische Expression einer von ihm kontrollierten kodierenden Nucleotidsequenz in Pflanzen bewirkt.

Somit betrifft die vorliegende Erfindung ein Nucleinsäuremolekül mit der Funktion eines karyopsenspezifischen Promotors, das
a) die durch die Nukleotide 1-4683 der Seq ID No. 1 definierte entsprechend der durch DSM 14224 (Plasmid p 15/G) hinterlegten Nucleinsäuresequenz umfaßt;
b) ein oder mehrere Sequenzelemente umfaßt, ausgewählt aus der Gruppe bestehend aus
   i) (Seq ID No. 2);
   ii) (Seq ID No. 3);
   iii) (Seq ID No. 4);
   iv) (Seq ID No. 5);
   v) (Seq ID No. 6);
   vi) (Seq ID No. 7);
   vii) (Seq ID No. 8);
   viii) (Seq ID No. 9) und
   ix) (Seq ID No. 10);
c) einen funktionalen Teil der in a) genannten Nucleinsäuresequenz umfaßt;
d) eine Sequenz umfaßt, die mit mindestens einer der in a) und/oder b) genannten Nucleinsäuresequenzen hybridisiert; und/oder
e) eine Sequenz umfaßt, die mit einer der in a) genannten Nucleinsäuresequenzen zu mindestens 60 %, vorzugsweise zu mindestens 75 %, insbesondere zu mindestens 90 % und ganz besonders bevorzugt zu mindestens 95% identisch ist.

Seq. ID No. 1 lautet:
tctagagaggtcacccgtcagtctatcctaagcgtgaaggggtcatgagccaatcactctaagcactcctgcacgtggcgcg
actggtcggggaccaagcccacctctatatacacagcaggcatgccgctcaccccaacaatcagcccgcagtctgtactgt
gacatcaggcagagctttcgggaggaactgacgacgctgaggggcccatacaccataatcccacggggtgattagtgtgt
atatgccagtgacagtctcagatcaaatactcaaatcttgttgagcgtgttattaagaaataaccttggacatcgaccagggcc
caggcccacttctctcctaggtggtctctacctgccttgtcgttccgccacgttgaatcactcgaggctgtcgggaacccaggc
ctatcactacctagatggtaccatctattccttcagcccttagttcgaacattatcataagtattacgttattatatagtatatctgtga
tcattggccaaagagaccacggctcaataatgtagcaatgcaaacggtgagactctagcagacaactaacatttatttacttt
gcagcgaagcacgggtgattcaagatagttctaatttttttaaagacggttctaattctttttttacggcaacacggttctaattcta
ccgttgcaacgcacaaggagatgtgctggtctctaacaatgtatgtaggagtttttttgttgcatggatcggacggttgaagatcg
taatataagtcacctttgacggtcgggaaaatggcggttatttctgtgttttcagacggctgacgcctggcaatcaccccaaaa
atatttttgtatgcgaggaggatcacctgccgccggctgacatccgccacatcagtaggttaggccaactcctccgcttgccac
cgaattaagctcgctgaaaagttcccctcccgacgcttcgcaggtaggtaggtgcatccatccccaactccccggccgtgcc
gcacacccccatctatatatgcaaatccagtccattcctgatcaaccaggacttgattagtagagcaagaggcctgaacaag
cacgcgctcgcagatcatcgacatgggttgtgagaggacgccgctggccgttgctctggcactggccctgctcctgggcctc
gcccacggcgacgtggtgcagttcatcttcggcgactcgctgtcggacgtgggcaacaacaactacctgaccaagagcctc
gcgcgcgcggcgctgccgtggtacggcatcgacttcggcagcggcatgcccaacggcaggttctgcaacggccgcaccg
tcgcggacatcatcggcgacaagatgggcctcccgcgcccgcccgcgttcctggacccgtccgtggacgagaccgtcatc
gccaagagcggcctcaactacgcgtccggcggcggcggcatcctcaacgagacctcgtccctcttcgtaagacacccatc

catcacttcaccaacttctcgtagctagacagcatggtagtatcatgagacatgaacgctccggttcgatcatcgcatctgact
gagacccatggcgcatgcatttgcagatccagaggttctcgctgtacaagcagatcgagctgttccaggggacgcaggcgt
tcatgcgggagaagatcgggcgggcggcggacaagctgttcggcgaggcctactacgtggtggccatgggcgcca
acgacttcatcaacaactacctgctccccgtctactccgactcgtggacctacaacggcgacaccttcgtcaagtacatggtc
accaccctggaggcccagctccggctcctgcacgggctgggcgcgcgccgggtcaccttcttcgggctggggcccatggg
ctgcatcccgctgcagcggctcctgcagaggtcctccacggcgtgccaggagtccaccaacaagctcgccctcagcttcaa
caagcaggccggcgcggtgatcagggagctggcggcgtcgctgcccaacgccacgttccagttcggggacgtctacgac
tacttccaggacatcatcgaccgcccctacatgcacggcttcaacaactcccacgcgccctgctgcacgctcggcaaggtg
cggccgaccctgacgtgcaccccgctctccacgctctgcaaggaccgcagcaagtacgtgttctgggacgagtaccaccc
caccgacagggccaacgagctcatcgcgctcgagacgctcaagcggctcaacatcaccgtcgttgccaacaccacctcc
agctagcctgcctgcctgccaccgacgccgcccaccaaaatgcgtacgcttcgacatgcatgggcgctgctgctgtgtgttgt
cttaattatactgcgggtgcttcgattgtaaccaaagtaggatgatcgaaaattctaggatgatgtccaagaaatgggatgga
gaatagatgcatgtacgtgtcctggatatgaaatttttttgagtatgagagaacagcataccaggatcatgcatctatcttaaatc
tcaagaggccactattaagacgttgatgtttaagacggtgatgttctatttgcatgtgaaatttcaagttcaaagacggtaccattt
atgagctatggaatcagccatgaatagtgatgtttactgttgacactattcattgctgcttttgtcttttggtaatgtgtttgaacttgga
aatttcacatactaatagaacatcacactcttaagacgtaatatttctttgagattttattttgaaacttcgcctgaagggtgctgat
gtgcccgctattcatctaggagactaggaaaatatatgcaaaaaaattcatacatatttaaaaatgataaatatgtatagaga
aaatgtttatcaactatagaaaaatatatgcaaaaaatataaatatgtatgaatttttttagcaagtatttaaatctagcatttgaa
agaaaaataaacaagtattagaaaaatgttaaacgtgtatagaaaaatgttaccatgtaattaaaaattgtataaaattatcat
gtatttttaaaaaaataaccaagcatttaaaaacaaatatttaaaaatgttaataaaggatttgaaaaattctaaacgtgtatac
aaaaatgttgaccatgtattaaaaaatgttaatcttgtatttaaaaatgtaatcaagcatttagaaaaacagttaaattgtataga
aatgtacccagaaaatcttgatattatatttcaaaaatgtaatcaagcatttgaaaaatatttaaaaatgtgtatagaaaaaatg
ttaaccatgtatttaaaaaattttaaacttgtatttgaaacatgttaatcatgtattagatatataccaaatatgtatgtaaaataaca
atgaaaatccaagggaaacgaaagaaaaacaaatgaaaacgggaaaaaaacaaaaaatgaaggaaaaaaaaga
aaaaacattgaaaaccaagaaagaaacaaagagaaccggagaataacaaacaaaagggaaagaaaaggtgaaaa
aactagtaaaaacaagaaacaaagaaaaaaggatgacaaacaaggaaaaaaattaaaaatccggaaaggcaacgg
taagacgactcttttccttcaagttggtagcgccctaccagggtaacacgaacttgacgatgactttatggctaggagagctac
gctggaacgaggagatccggaccaaaccatgtgcgctacaaaagtgtattattatttttgcaaaaatgatccgaatctattat
caaaattcagcgaaatacaaaacatctcgaacataatgaacaatacattgagattccaggaccccaaacaaccactactg
ccgcgaagaaaaaggattgggaggacagaaattatcctaaccacgttcgtcctcggttgttggtctcatcgcgcgctaaac
aacctggacaacagaaaaggcaaagcagtgtcctccgctccgcagcaaagaagacaaatcgtcacttgtcagaggccgt
cacccaagcaagcaaactgcaaagcttgttcgtttggtttatcccgtagtacgcgccaacgcatgtgccgcaccgcgtttgcg
gtggagagcgcaggcatgcatcaaccaacaaacgaaacagtgcagttgcttacagtgctccatccctccaaaaaaaaa
gttgcagtgctctatctatctatctacacaatcaacgcgggcctcctgctccttcgccgcaagccccgttccgtcctcagtcttca
cgtggattctgcaacctccttccagcagcttgtcaccacggacgcttcctcgtgcgctgctcgcgtggcaccggccccgctttc
cagcgtgctccgcgcgggccgcggccgcaaatcgcagacccaacacgccacccgccagggggccgttcgtacgtaccc
gcccctcgtgtaaagccgccgccgtcgtcgccgtcccccgctcgcggccatttccccggcctgaccccgtgcgtttaccccac
agagcacactccagtccagtccagcccactgccgccgcgctactccccactcccgctgccaccacctccgcctgcgccgc
gctctgggcggaggaccaacccgcgcatcgtaccatcgcccgccccgatcccggccgccgccATGTCGTCGGCG
GTCGCGTCCGCCGCGTCCTTCCTCGCGCTCGCCTCCGCCTCCCCGGGAGATCAC

GCAGGCGGGCGAGGGTGAGCGCCGCCACCCCACGCCGGGCCGGCAGGCTC
CACTGGCCGCCGTGGCCGCCGCAGCGCACGGCTCGCGACGGAGGTGTGGCCGCC
CGCGCCGCCGGGAAGAAGGACGCGAGGGTCGACGACGACGCCGCGTCCGCGAG
CAGCCCCGCGCACGCCGCGGTGGCGCCGCCACCAAGgtagttggttcgttatgacttgctgtatgg
gcgtgcgcctcgagatcagctcacgaattgtttctacaaaacgcacgcgctcgtgtgcagGTCGCGGAGCGGAG
GGATCC

Weiterhin ist Gegenstand der vorliegenden Erfindung ein Nucleinsäuremolekül mit der Funktion eines karyopsenspezifischen Promotors, das
a) ein oder mehrere Sequenzelemente umfaßt, ausgewählt aus der Gruppe bestehend aus
   i) 
tctagagaggtcacccgtcagtctatcctaagcgtgaaggggtcatgagccaatca
ctctaagcactcctgcacgtggcgcgactggtcggggaccaagcccacctctatat
acacagcaggcatgccgctcaccccaacaatcagcccgcagtctgtactgtgac
atcaggcagagctttcgggaggaactgacgacgctgaggggcccatacaccata
atcccacggggtgattagtgtgtatatgccagtgacagtctcagatcaaatactcaa
atcttgttgagcgtgttattaagaaataaccttggacatcgaccagggcccaggccc
acttctctcctaggtggtctctacctgccttgtcgttccgccacgttgaatcactcgagg
ctgtcgggaacccaggcctatcactacctagatggtaccatctattccttcagcccctt
agttcgaacattatcataagtattacgttattatatagtatatctgtgatcattggccaaa
gagaccacggctcaataatgtagcaatgcaaacggtgagactctagcagacaac
taacatttatttactttgcagcgaagcacgggtgattcaagatagttctaatttttttaaa
gacggttctaattctttttttacggcaacacggttctaattctaccgttgcaacgcaca
aggagatgtgctggtctctaacaatgtatgtaggagtttttgttgcatggatcggacg
gttgaagatcgtaatataagtcacctttgacggtcgggaaaatggcggttatttctgtg
ttttcagacggctgacgcctggcaatcaccccaaaaat (Seq ID No. 2);
   ii) 
atttttgtatgcgaggaggatcacctgccgccggctgacatccgccacatcagtag
gttaggccaactcctccgcttgccaccgaattaagctcgctgaaaagttcccctccc
gacgcttcgcaggtaggtaggtgcatccatccccaactccccggccgtgccgcac
acccccatctatatatgcaaatccagtccattcctgatcaaccaggacttgattagta
 
gagcaagaggcctgaacaagcacgcgctcgcagatcatcgacatgggttgtgag
aggacgccgctggccgttgctctggcactggccctgctcctgggcctcgcccacgg
cgacgtggtgcagttcatcttcggcgactcgctgtcggacgtgggcaacaacaact
acctgaccaagagcctcgcgcgcgcggcgctgccgtggtacggcatcgacttcg
gcagcggcatgcccaacggcaggttctgcaacggccgcaccgtcgcggacatc
atcggcgacaagatgggcctcccgcgcccgcccgcgttcctggacccgtccgtgg
acgagaccgtcatcgccaagagcggcctcaactacgcgtccggcggcggcggc
atcctcaacgagacctcgtccctcttcgtaagacacccatccatcacttcaccaactt
ctcgtagctagacagcatggtagtatcatgagacatgaacgctccggttcgatcatc
gcatctgactgagacccatggcgcatgcatttgcagatccagaggttctcgctgtac
aagcagatcgagctgttccaggggacgcaggcgttcatgcgggagaagatcgg
gcgggcggcggcggacaagctgttcggcgaggcctactacgtggtggc (Seq
ID No. 3);
   iii) 
catgggcgccaacgacttcatcaacaactacctgctccccgtctactccgactcgtg
gacctacaacggcgacaccttcgtcaagtacatggtcaccaccctggaggccca
gctccggctcctgcacgggctgggcgcgccgggtcaccttcttcggctgggg
cccatgggctgcatcccgctgcagcggctcctgcagaggtcctccacggcgtgcc
aggagtccaccaacaagctcgccctcagcttcaacaagcaggccggcgcggtg
atcagggagctggcggcgtcgctgcccaacgccacgttccagttcggggacgtct
acgactacttccaggacatcatcgaccgcccctacatgcacggcttcaacaactcc
cacgcgccctgctgcacgctcggcaaggtgcggccgaccctgacgtgcaccccg
ctctccacgctctgcaaggaccgcagcaagtacgtgttctgggacgagtaccacc
ccaccgacagggccaacgagct (Seq ID No. 4);
   iv) 
catcgcgctcgagacgctcaagcggctcaacatcaccgtcgttgccaacaccacc
tccagctagcctgcctgcctgccaccgacgccgcccaccaaaatgcgtacgcttc
gacatgcatgggcgctgctgctgtgtgttgtcttaattatactgcgggtgcttcgattgt
aaccaaagtaggatgatcgaaaattctaggatgatgtccaagaaatgggatgga
gaatagatgcatgtacgtgtcctggatatgaaattttttttgagtatgagagaacagca
taccaggatcatgcatctatcttaaatctcaagaggccactattaagacgttgatgttt
aagacggtgatgttctatttgcatgtgaaatttcaagttcaaagacggtaccatttatg
 
agctatggaatcagccatgaatagtgatgtttactgttgacactattcattgctgcttttg
tcttttggtaatgtgtttgaacttggaaatttcacatacta (Seq ID No. 5);
   v) 
atagaacatcacactcttaagacgtaatatttctttgagattttatttttgaaacttcgcct
gaagggtgctgatgtgcccgctattcatctaggagactaggaaaatatatgcaaaa
aaattcatacatatttaaaaatgataaatatgtatagagaaaatgtttatcaactatag
aaaaatatatgcaaaaaatataaatatgtatgaatttttttagcaagtatttaaatctag
catttgaaagaaaaataaacaagt (Seq ID No. 6);
   vi) 
attagaaaaatgttaaacgtgtatagaaaaatgttaccatgtaattaaaaattgtata
aaattatcatgtatttttaaaaaaataaccaagcatttaaaaacaaatatttaaaaat
gttaataaaggatttgaaaaattctaaacgtgtatacaaaaatgttgaccatgtatta
aaaaatgttaatcttgtatttaaaaatgtaatcaagcatttagaaaaacagttaaattg
tatagaaatgtacccagaaaatcttgatattatatttcaaaaatgtaatcaagcatttg
aaaaatattttaaaaatgtgtatagaaaaaatgtt (Seq ID No. 7);
   vii) 
aaccatgtatttaaaaaattttaaacttgtatttgaaacatgttaatcatgtattagatat
ataccaaatatgtatgtaaaataacaatgaaaatccaagggaaacgaaagaaa
aacaaatgaaaacgggaaaaaaacaaaaaatgaaggaaaaaaaagaaaaa
acattgaaaaccaagaaagaaacaaagagaaccggagaataacaaacaaaa
gggaaagaaaaggtgaaaaaa (Seq ID No. 8);
   viii) 
ctagtaaaaacaagaaacaaagaaaaaaggatgacaaacaaggaaaaaaat
taaaaatccggaaaggcaacggtaagacgactcttttccttcaagttggtagcgcc
ctaccagggtaacacgaacttgacgatgactttatggctaggagagctacgctgg
aacgaggagatccggaccaaaccatgtgcgctacaaaagtgtattattatttttgc
aaaaatgatccgaatctattatcaaaattcagcgaaatacaaaacatctcgaacat
aatgaacaatacattgagattccaggaccccaaacaaccactactgccgcgaag
aaaaaaggattgggaggacagaaattatcctaaccacgttcgtcctcggttgttggt
ctcatcgcgcgctaaacaacctggacaacagaaaaggcaaagcagtgtcctcc
gctccgcagcaaagaagacaaatcgtcacttgtcagaggccgtcacccaagca
agcaaactgcaa (Seq ID No. 9) und
   ix)

agcttgttcgtttggtttatcccgtagtacgcgccaacgcatgtgccgcaccgcgtttg
cggtggagagcgcaggcatgcatcaaccaacaaacgaaacagtgcagttgctta

cagtgctccatccctccaaaaaaaaaagttgcagtgctctatctatctatctacaca
atcaacgcgggcctcctgctccttcgccgcaagccccgttccgtcctcagtcttcac
gtggattctgcaacctccttccagcagcttgtcaccacggacgcttcctcgtgcgctg
ctcgcgtggcaccggccccgctttccagcgtgctccgcgcgggccgcggccgca
aatcgcagacccaacacgccacccgccagggggccgttcgtacgtacccgccc
ctcgtgtaaagccgccgccgtcgtcgccgtcccccgctcgcggccattt (Seq ID
No. 10);

   sowie
b) einen funktionalen Teil der Seq ID No. 1 umfaßt, vorzugsweise ein oder mehrere Sequenzelemente aus der Gruppe bestehend aus den Nukleotiden der Position 1-72, 78-194, 200-402, 409-579, 588-734, 743-837, 852-865, 872-940, 948-957, 962-1113, 1120-1180, 1186-1218, 1227-1330, 1336-1538, 1545-1567, 1574-1589, 1597-2015, 2021-2043, 2052-2087, 2096-2276, 2292-2320, 2336-2352, 2361-2470, 2478-2531, 2540-2602, 2609-2712, 2721-2786, 2794-2870, 2883-2937, 2943-2979, 2986-3048, 3056-3073, 3080-3098, 3106-3133, 3142-3155, 3163-3197, 3205-3289, 3302, 3311-3317, 3318-3405, 3414-3446, 3453-3533, 3541-3570, 3578-3617, 3625-3750, 3757-3978, 3988-4031, 4038-4109, 4116-4145, 4153-4173, 4180-4294, 4301-4419, 4427-4449, 4456-4466, 4474-4480, 4489-4683 der Seq. ID No. 1.

Im Rahmen der vorliegenden Erfindung werden die Begriffe "erfindungsgemäßes Nukleinsäuremolekül" und "erfindungsgemäßer Promotor" als Synonyme verwendet.
In einer bevorzugten Ausführungsform sind die erfindungsgemäßen Promotoren solche von pflanzlichen Genen, vorzugsweise von monokotylen Pflanzen, oder von solchen Genen abgeleitet. In einer weiteren, bevorzugten Ausführungsform sind die erfindungsgemäßen Promotoren zur Expression oder Suppression von Genen in genetisch modifizierten Organismen, vorzugsweise in genetisch modifizierten Pflanzen, Algen oder Hefen, speziell in genetisch modifizierten monokotylen Pflanzen und insbesondere zur Expression oder Suppression von Stärkesynthase-Genen in besagten genetisch modifizierten Organismen geeignet. Die erfindungsgemäßen Promotoren können hierbei aus pflanzlichen Genen stammen, aus Algen oder Hefen gewonnen werden, durch rekombinante DNA-Techniken modifiziert sein und/oder synthetisch hergestellt werden.

Die erfindungsgemäßen Promotoren können z.B. modifiziert werden, indem sie mit weiteren *cis*-regulatorischen Elementen kombiniert werden. So können die erfindungsgemäßen Promotoren zusätzlich mit Enhancer-Elementen kombiniert werden, um die Expression des korrespondierenden Nucleinsäuremoleküls zu verstärken, ohne jedoch seine gewebespezifische Expression zu beeinflussen. Auch individuelle *cis*-Elemente der isolierten Promotoren (siehe unten) können ebenfalls zu regulatorischen Einheiten miteinander kombiniert werden.

Ein Maß für die Promotoraktivität ist beispielsweise die für ein bestimmtes Markergen ermittelte Expressionsrate, welches unter der regulativen Kontrolle des erfindungsgemäßen Promotors steht. Beispiele für geeignete Markergene sind das β-Glucuronidase-Gen (gus) aus *E*. *coli* (Jefferson (1987) Plant Molecular Biology Reporter Vol.5 (4): 387-405) oder das Green-Fluorescence-Protein-Gen (gfp) (Baulcombe et al., Plant J. 7 (16) (1993), 1045-1053). Die Organ- bzw. Gewebespezifität läßt sich leicht durch Vergleich der an einzelnen Geweben bzw. Organen der Pflanze ermittelten Expressionsraten für besagte Markergene bestimmen. Funktionale Teile der erfindungsgemäßen Promotorsequenz umfassen im Rahmen der vorliegenden Erfindung sowohl natürlich vorkommende Varianten, als auch künstliche, z.B. durch Mutagenese oder chemische Synthese erhaltene Nucleotidsequenzen.

Im Zusammenhang mit der vorliegenden Erfindung wird unter einem "Promotor" eine DNA-Sequenz verstanden, die den regulatorischen Anteil eines Gens, vorzugsweise eines Strukturgens, umfaßt. Unter dem "regulatorischen Anteil" eines Gens wird derjenige Anteil verstanden, der die Expressionsbedingungen des Gens bestimmt. Der regulatorische Anteil besitzt Sequenzmotive, mit denen Transkriptionsfaktoren und RNA-Polymerase(n) in Wechselwirkung treten und die Transkription des kodierenden Anteils des Gens einleiten. Darüber hinaus kann der regulatorische Anteil ein oder mehrere positive regulatorische Elemente, sogenannte 'Enhancer' umfassen. Er kann zusätzlich oder an deren Stelle aber auch negativ regulatorische Elemente, sogenannte 'Silencer', enthalten. Unter einem "Strukturgen" wird im allgemeinen eine genetische Einheit aus regulatorischem und kodierendem Anteil verstanden, deren Genprodukt im allgemeinen ein Protein ist. Die Information für die primäre Aminosäuresequenz des Genprodukts ist im kodierenden Anteil des Strukturgens enthalten, während der regulatorische Anteil bestimmt, wann, in welchen Geweben, unter welchen physiologischen Bedingungen und in welchen Mengen das Transkript des kodierenden Anteils gebildet wird, nach dessen Vorlage das Genprodukt synthetisiert wird.

Unter dem Begriff "karyopsenspezifisch" versteht man im Rahmen der vorliegenden Erfindung, daß ein unter der Kontrolle eines erfindungsgemäßen Promotors stehendes Gen in der Karyopse, d.h. Endosperm, Perikarp, Aleuron, Embryo und/oder Scutellum exprimiert wird, vorzugsweise zu einem frühen Zeitpunkt, d.h. früher als 15 dap (dap = Tage nach der Befruchtung), vorzugsweise früher als 10 dap, besonders bevorzugt früher als 6 dap. Für besagte "frühe Expression" gilt im Sinne der vorliegenden Erfindung eine Untergrenze für den Expressionszeitpunkt von vorzugsweise 5 dap, besonders bevorzugt von 3 dap, insbesondere von 2 dap und ganz besonders bevorzugt von 1 dap. Speziell ist Karyopsenspezifität im Rahmen der vorliegenden Erfindung dann gegeben, wenn der erfindungsgemäße Promotor die Expression eines Gens in der Karyopse, vorzugsweise im zentralen Stärkeendosperm im Vergleich zu anderen Geweben wie z.B. maturen Blättern oder Wurzeln begünstigt und in der Karyopse eine signifikante Erhöhung, d.h. mindestens 2- bis 5-fache, vorzugsweise 5- bis 10-fache, insbesondere 10- bis 100-fache oder höhere Expression bewirkt.

Im Zusammenhang mit der vorliegenden Erfindung kann Karyopsenspezifität z.B. durch übliche Reportergen-Experimente analysiert werden. Zur Testung einer isolierten Promotorsequenz auf deren Promotoraktivität in Karyopse kann der Promotor beispielsweise in einer Expressionskassette bzw. in einen Vektor zur Pflanzentransformation operativ mit einem Reportergen, wie z.B. dem β-Glucuronidasegen aus *E. coli*, verknüpft werden. Dieses Konstrukt wird dann zur Transformation von Pflanzen verwendet. Anschließend wird die Expression der β-Glucuronidase in der Karyopse im Vergleich zu anderen Geweben, wie z.B. maturen Blättern oder Wurzeln bestimmt, wie z.B. bei Martin et al. (The GUS Reporter System as a Tool to Study Plant Gene Expression, In: GUS Protocols: Using the GUS Gene as a Reporter of Gene Expression, Academic Press (1992), 23-43) beschrieben.

Der Begriff "Karyopse" ist dem Fachmann geläufig und umfaßt insbesondere Perikarp und Endosperm. Da diese Gewebe eine dynamische Entwicklung durchlaufen, korreliert z.B. die Entwicklung des Endosperms in verschiedene Zell- oder Gewebetypen mit unterschiedlichen biochemischen Aktivitäten, bedingt durch eine differenzielle Genexpression. Ergänzend sei auf Olsen et al. verwiesen (Olsen et al., 1999, Trends in Plant Science 4 (7), 253-257).

Der erfindungsgemäße Promotor erlaubt eine karyopsenspezifische Genexpression einer von ihm kontrollierten kodierenden Nucleotidsequenz. Er stellt eine interessante Alternative zu den bekannten endosperm-spezifischen Promotoren dar, weil er zu einem sehr frühen Zeitpunkt in der Karyopse aktiv ist, d.h. <15 dap, vorzugsweise <10 dap, insbesondere <6 dap (dap = Tage nach der Bestäubung). Mit Hilfe des erfindungsgemäßen Promotors kann besonders die Expression von Genen effektiv gesteuert werden, deren Genprodukte am Stärkemetabolismus in monokotylen Pflanzen und insbesondere in Weizen beteiligt sind.

Vielfältige Verwendungsmöglichkeiten stehen darüber hinaus für die erfindungsgemäßen Promotoren zur Verfügung. Beispielsweise wird die Herstellung transgener Pflanzen ermöglicht, die aufgrund eines modifizierten Metabolismus in der Karyopse eine qualitativ und/oder quantitativ veränderte Speicherstoffzusammensetzung in ihrem Speichergewebe, d.h. im Getreidekom aufweisen.
Neben dem Promotor, der die gesamte durch die Nukleotide 1 4683 der SEQ ID No. 1 definierten Sequenz, bzw. der entsprechend durch DSM 14224 hinterlegten Sequenz aufweist, betrifft die vorliegende Erfindung auch Promotoren, die einen funktionalen Teil dieser Sequenz aufweisen und die in Pflanzen eine karyopsenspezifische Expression einer von ihnen kontrollierten kodierenden Nucleotidsequenz bewirken.

Unter einem "funktionalen Teil" des erfindungsgemäßen Promotors versteht man im Rahmen der vorliegenden Erfindung solche Sequenzen, welche nicht die vollständige Sequenz des Promotors wie durch Nukleotid 1-4683 der SEQ ID No. 1 definiert, bzw. durch DSM 14224 hinterlegt, umfassen, sondern verkürzt sind. Trotz der Verkürzung weist ein "funktionaler Teil des erfindungsgemäßen Promotors" die erfindungsgemäße Karyopsenspezifität auf. Sequenzen die einen funktionalen Teil des erfindungsgemäßen Promotors der Seq. ID No. 1 enthalten, weisen vorzugsweise einen oder mehrere der nachfolgend aufgezählten Abschnitte aus der SEQ ID No. 1 auf: 1-72, 78-194, 200-402, 409-579, 588-734, 743-837, 852-865, 872-940, 948-957, 962-1113, 1120-1180, 1186-1218, 1227-1330, 1336-1538, 1545-1567, 1574-1589, 1597-2015, 2021-2043, 2052-2087, 2096-2276, 2292-2320, 2336-2352, 2361-2470, 2478-2531, 2540-2602, 2609-2712, 2721-2786, 2794-2870, 2883-2937, 2943-2979, 2986-3048, 3056-3073, 3080-3098, 3106-3133, 3142-3155, 3163-3197, 3205-3289, 3302, 3311-3317, 3318-3405, 3414-3446, 3453-3533, 3541-3570, 3578-3617, 3625-3750, 3757-3978, 3988-4031, 4038-4109, 4116-4145, 4153-4173, 4180-4294, 4301-4419, 4427-4449, 4456-4466, 4474-4480, 4489-4683. Die angegebenen Ziffern bedeuten die Nukleotidpositionen in der Seq. ID No. 1.

Unter einem "funktionalen Teil" der erfindungsgemäßen Promotorsequenz werden insbesondere auch natürliche oder künstliche Mutationen einer ursprünglich erhältlichen Promotorsequenz verstanden, welche die erfindungsgemäßen Merkmale aufweisen. Der Begriff "Mutation" umfasst hierbei die Substitution, Addition, Deletion, Vertauschung und/oder Insertion eines oder mehrerer Nucleotide bzw. Nukleotidmotive, insbesondere von sog. "cis-Elementen" (siehe unten). Zweck solcher Modifikationen kann z.B. die Herstellung von Fragmenten, die Einführung oder Umstellung von bekannten Nukleotid-Motiven wie z.B. von Restriktionsschnittstellen oder cis-Elementen sein. Somit werden beispielsweise auch solche Nucleotidsequenzen durch die vorliegende Erfindung umfaßt, welche durch Modifikation der durch Die Nukleotide 1-4683 der Seq ID No. 1 definierten bzw. der durch DSM 14224 hinterlegten Promotorsequenz erhältlich sind und die erfindungsgemäß wesentlichen strukturellen und funktionellen Merkmale aufweisen.

"Funktionale Teile" der erfindungsgemäßen Promotorsequenz umfassen dabei auch solche Promotorvarianten, deren Promotoraktivität, verglichen mit dem unmodifizierten, d.h. natürlich erhältlichen Promotor (Wildtyp), abgeschwächt oder verstärkt ist.

Insbesondere werden unter einem "funktionalen Teil" der erfindungsgemäßen Promotorsequenz die durch Deletionsanalyse (vgl. Beispielteil) identifizierbaren Bereiche verstanden, vorzugsweise die Sequenzabschnitte 2241-4683; 2637-4683; 3569-4683; 4071-4683; 4151-4683 und 4403-4683 der Seq ID No. 1.

Prinzipiell wird die Aktivität eines eukaryontischen RNA-Polymerase II-Promotors durch das synergistische Zusammenwirken verschiedener *trans*-aktiver Faktoren (DNA-bindende Moleküle wie Proteine oder Hormone) bedingt, welche an die verschiedenen im Promotor vorhandenen *cis*-regulatorischen DNA-Elemente ('cis-Elemente') binden, in der Regel DNA-Bereiche von etwa 10-20 Nukleotiden Länge. Diese Faktoren wechselwirken direkt oder indirekt mit einzelnen oder mehreren Faktoren der basalen Transkriptionsmaschinerie, was letztlich zur Ausbildung eines Präinitiationskomplexes in der Nähe der Transkriptionsstartstelle führt (Drapkin et al., Current Opinion in Cell Biology 5 (1993), 469-476). Man kann von einem modularen Aufbau eukaryontischer RNA-Polymerase II-Promotoren ausgehen, wobei die *cis-*Elemente (Module) als Teilkomponenten des Promotors dessen Aktivität im einzelnen determinieren (Tjian und Maniatis, Cell 77 (1994), 5-8).

Einzelne, potentiell Gewebespezifität vermittelnde Subdomänen des erfindungsgemäßen Promotors können beispielsweise durch Fusion mit einer Minimalpromotor-Reportergen-Kassette identifiziert werden. Unter einem Minimalpromotor versteht man eine DNA-Sequenz, die eine TATA-Box, die sich etwa 20 bis 30 Basenpaare stromaufwärts von der Transkriptionsstartstelle befindet, oder eine Initiatorsequenz (Smale und Baltimore, Cell 57 (1989), 103-113; Zawel und Reinberg, Proc. Natl. Acad. Sci. 44 (1993), 67-108; Conaway und Conaway, Annu. Rev. Biochem 62 (1993), 161-190) umfaßt. Beispiele für Minimalpromotoren sind der -63 bis +8 Δ35S-Promotor (Frohberg, Dissertation an der Freien Universität Berlin, Fachbereich Biologie (1994)), der -332 bis +14 Minimal-Patatin-Classl-Promotor sowie der -176 bis +4-Minimal-PetE-Promotor (Pwee et al., Plant J. 3 (1993), 437-449.)

Desweiteren können Subdomänen bzw. *cis*-Elemente des erfindungsgemäßen Promotors auch über Deletionsanalysen bzw. Mutagenese identifiziert werden (Kawagoe et al., Plant J. 5(6) (1994), 885-890). Der Test auf Funktionalität einer solchen Subdomäne oder *cis-*Elements des Promotors kann *in planta* durch den Nachweis von Reportergenaktivität in stabil transformierten Zellen erfolgen.

In einer weiteren Ausführungsform betrifft die vorliegende Erfindung daher insbesondere durch Di- oder Multimerisierung von Subdomänen bzw. *cis-*Elementen von SEQ ID No.1 erhaltenen Modifikationen von Seq. ID No. 1, insbesondere der Nukleotidsequenz 1-4683 der Seq ID No. 1.

In einer weiteren Ausführungsform der Erfindung wird die Erhöhung der Promotoraktivität im Vergleich zum Wildtyp durch die Kombination des erfindungsgemäßen Promotors mit einem oder mehreren 'Enhancern' erreicht. In der Literatur sind verschiedene Enhancer-Elemente beschrieben worden, die in der Regel eine gewebespezifische Erhöhung der Expression bewirken, wobei die Gewebespezifität im allgemeinen durch den jeweils verwendeten Enhancer bestimmt wird (Benfey et al., Science 250 (1990), 959-966; Benfey et al., EMBO J. 8 (1989), 2195-2202; Chen et al., EMBO J. 7, (1988), 297-302; Simpson et al., Nature 323 (1986), 551-554).

Darüberhinaus gibt es auch Enhancer-Elemente, wie z.B. den PetE Enhancer (Sandhu et al., Plant Mol. Biol. 37 (1998), 885-896), die nicht gewebespezifisch wirken und somit als quantitative Verstärkerelemente vor den erfindungsgemäßen Promotor gesetzt werden können, um die Expressionsrate des kontrollierten Genes in der Karyopse zu erhöhen, ohne die Gewebespezifität des erfindungsgemäßen Promotors zu verändern.

Ferner können auch die dem Fachmann bekannten synthetischen Enhancer-Elemente verwendet werden, die beispielsweise von natürlich vorkommenden Enhancem abgeleitet sind und/oder durch Kombination von Enhancer-Elementen erhalten werden.

Ebenso betrifft die vorliegende Erfindung auch Promotoren, die eine Nucleotidsequenz aufweisen, die mit der durch Die Nukleotide 1-4683 der SEQ ID No. 1 definierten bzw. durch DSM 14224 hinterlegten Nucleotidsequenz hybridisieren, vorzugsweise unter stringenten Bedingungen und die in Pflanzen einen karyopsenspezifischen Einfluß auf die Expression einer von ihnen kontrollierten codierenden Nucleotidsequenz ausüben.

Der Ausdruck "stringente Bedingungen" bedeutet dabei beispielsweise Hybridisierungsbedigungen, wie sie in Sambrook et al. (Molecular Cloning, A Laboratory Manual, 2. Aufl. (1989), Cold Spring Harbor Laboratory Press, Cold Spring Harbor, NY) beschreiben sind. Insbesondere findet eine stringente Hybridisierung unter den folgenden Bedingungen statt: Hybridisierungspuffer: 2x SSC; 10x Denhardt's Lösung (Fikoll 400 + PEG + BSA; Verhältnis 1:1:1); 0.1% SDS; 5 mM EDTA; 50 mM Na₂HPO₄; 250 µg/ml Heringsperma-DNA; 50 µg/ml tRNA; oder 0.25 M Natriumphosphatpuffer pH 7.2, 1 mM EDTA, 7% SDS Hybridisierungstemperatur T= 65 bis 68 °C; Waschpuffer 0.2 x SSC; 0.1 % SDS; Waschtemperatur T = 65 bis 68° C.

Vorzugsweise weisen derartige Promotoren eine Sequenzidentität von mindestens 30%, speziell bevorzugt von mindestens 40%, sehr bevorzugt von mindestens 50%, besonders bevorzugt mindestens 60%, insbesondere bevorzugt von mindestens 70%, ganz besonders bevorzugt von mindestens 80%, ganz besonders speziell bevorzugt von mindestens 90% und insbesondere ganz besonders speziell bevorzugt von mindestens 95% zu den Nukleotiden 1-4683 der Seq ID No. 1 oder erfindungsgemäß funktionalen Teilen davon auf.

Der Grad der Identität von Sequenzen mit dem erfindungsgemäßen Promotor kann durch üblichen Sequenzvergleich mit den Nukleotiden 1-4683 der SEQ ID No. 1 bestimmt werden. Wenn zwei zu vergleichende Sequenzen eine unterschiedliche Länge aufweisen, bezieht sich die Sequenzidentität vorzugsweise auf den prozentualen Anteil der Nucleotidreste der kürzeren Sequenz, die identisch sind mit den Nucleotidresten der längeren Sequenz. Die Sequenzidentität kann üblicherweise durch Verwendung von Computerprogrammen wie z. B. das Bestfit-Programm (Wisconsin Sequence Analysis Package, Version 8 for Unix, Genetics Computer Group, University Research Park, 575 Science Drive Madison, WI 53711) bestimmt werden. Bestfit nützt den lokalen Homologiealgorithmus von Smith und Waterman, Advances in Applied Mathematics 2 (1981), 482-489, um das Segment mit höchster Sequenzidentität zu bestimmen. Bei der Anwendung von Bestfit oder einem anderen Sequenz-Alignment-Programm zur Bestimmung, ob eine bestimmte Sequenz beispielsweise zu 95% identisch ist mit einer Referenzsequenz der vorliegenden Erfindung, werden die Parameter vorzugsweise so eingestellt, daß der Prozentanteil der Identität über die gesamte Länge der Referenzesequenz berechnet wird und daß Homologielücken ("gaps") von bis zu 5% der Gesamtzahl der Nucleotide in der Referenzsequeriz erlaubt sind. Bei der Verwendung von Bestfit können die sogenannten optionalen Parameter bei ihren voreingestellten ("default") Werten belassen werden. Die Abweichungen, die bei dem Vergleich einer gegebenen Sequenz mit den oben beschriebenen Sequenzen der Erfindung auftreten, können beispielsweise durch Addition, Deletion, Substitution, Insertion oder Rekombination verursacht sein. Promotorsequenzen, die wie oben beschrieben mit den Nukleotiden 1-4683 der SEQ ID No. 1 bzw. der entsprechenden, durch DSM 14224 hinterlegten Nucleotidsequenz hybridisieren, stammen vorzugsweise aus pflanzlichen Organismen, bevorzugt aus höheren Pflanzen, besonders bevorzugt aus monokotylen Pflanzen, insbesondere bevorzugt aus Gramineen und ganz besonders bevorzugt aus Pflanzen der Gattung *Triticum.*

Ferner betrifft die vorliegende Erfindung auch Promotoren, die einen funktionalen Teil dieser Sequenzen aufweisen und die in Pflanzen eine karyopsenspezifische Expression einer von ihnen kontrollierten kodierenden Nucleotidsequenz bewirken und die ein oder mehrere der Sequenzen ausgewählt aus der Gruppe bestehend aus den Nukleotiden 1-4683 der Seq ID No. 1, Seq ID No. 2, Seq ID No. 3, Seq ID No. 4, Seq ID No. 5, Seq ID No. 6, Seq ID No. 7, Seq ID No. 8, Seq ID No. 9 und Seq ID No. 10 umfassen.

Die vorliegende Erfindung betrifft weiterhin Expressionskassetten enthaltend einen oder mehrere der erfindungsgemäßen Promotoren. Unter dem Begriff "Expressionskassette" wird hierbei die Kombination eines erfindungsgemäßen Promotors mit einer zu exprimierenden Nucleinsäuresequenz verstanden. Diese Nucleinsäuresequenz kann beispielsweise eine ein Polypeptid kodierende Sequenz sein, z.B. ein Gen, das in sense- oder in antisense-Orientierung mit dem Promotor verknüpft sein kann. Die Nucleinsäuresequenz kann auch eine nichttranslatierbare RNA kodieren, beispielsweise eine antisense-RNA oder ein Ribozym. Diese Nucleinsäuresequenzen können in Verbindung mit dem erfindungsgemäßen Promotor benutzt werden, um Pflanzen mit einem verändertem Phänotyp herzustellen.

Die erfindungsgemäßen Expressionskassetten können weiterhin eine Transkriptionsterminationssequenz stromabwärts des 3'-Endes der mit dem Promotor verknüpften Nucleinsäuresequenz enthalten. Unter einer "Transkriptionsterminationssequenz" wird dabei eine DNA-Sequenz verstanden, die am 3'-Ende eines kodierenden Genabschnitts lokalisiert ist und in der Lage ist, die Beendigung der Transkription und gegebenenfalls die Synthese eines Poly-A-Schwanzes hervorzurufen. Ein Beispiel für eine solche Terminationssequenz ist die des Octopinsynthasegens. Weitere sind dem Fachmann gut bekannt. Außerdem betrifft die vorliegende Erfindung einen Vektor, der eine oder mehrere erfindungsgemäße Promotoren bzw. Expressionskasetten enthält.

In einer weiterhin bevorzugten Ausführungsform ist der Promotor in dem erfindungsgemäßen Vektor mit Restriktionsschnittstellen bzw. einem Polylinker verknüpft, die eine Integration beliebiger Sequenzen stromabwärts des Promotors erlauben. Dabei wird unter einem "Polylinker" eine DNA-Sequenz verstanden, die Erkennungssequenzen von mindestens drei Restriktionsenzymen, vorzugsweise von 5 oder mehr Restriktionsenzymen, enthält.

In einer besonders bevorzugten Ausführungsform enthält der erfindungsgemäße Vektor zusätzlich eine Sequenz für die Termination der Transkription, beispielsweise die des Octopinsynthasegens, stromabwärts des Promotors bzw. des Polylinkers.

Somit betrifft die vorliegende Erfindung ebenfalls Vektoren, die eine oder mehrere erfindungsgemäße Expressionskassetten enthalten. Gegebenenfalls enthalten die erfindungsgemäßen Vektoren Selektionsmarker, die geeignet sind, Zellen, die die erfindungsgemäßen Vektoren enthalten, nach der Transformation leicht zu identifizieren und gegebenenfalls zu selektionieren.

In einer bevorzugten Ausführungsform sind die erfindungsgemäßen Vektoren geeignet zur Transformation von pflanzlichen Zellen und besonders bevorzugt zur Integration von Fremd-DNA (z.B. Transgenen) in das pflanzliche Genom. Ein Beispiel für derartige Vektoren sind binäre Vektoren, die zum Teil kommerziell erhältlich sind.

Ferner betrifft die vorliegende Erfindung Wirtszellen, die mit einem erfindungsgemäßen Nukleinsäuremolekül, bzw. erfindungsgemäßen Promotor, einer erfindungsgemäßen Expressionskassette oder einem erfindungsgemäßen Vektor genetisch modifiziert sind, insbesondere pflanzliche Zellen oder mikrobielle Zellen, z. B. der Gattung *Agrobacterium*.

"Genetisch modifiziert" bedeutet dabei, daß die Wirtszelle einen erfindungsgemäßen Promotor, eine erfindungsgemäße Expressionskassette oder einen erfindungsgemäßen Vektor enthält, vorzugsweise stabil in das Genom der Wirtszelle integriert, und der Promotor bzw. die Expressionskassette entweder in die Wirtszelle oder zuvor in einen Vorgänger dieser Zelle als Fremd-DNA eingebracht wurde. Die erfindungsgemäßen Wirtszellen können daher selbst das unmittelbare Produkt einer Transformation im Sinn der vorliegenden Erfindung sein, oder von solchen Zellen abstammen, die einen erfindungsgemäßen Promotor oder eine erfindungsgemäße Expressionskassette enthalten. Als Wirtszellen kommen sowohl prokaryontische, insbesondere bakterielle Zellen, als auch eukaryontische Zellen in Frage. Eukaryontische Zellen können pflanzlichen Ursprungs sein, aber auch von Pilzen stammen, insbesondere von der Gattung *Saccharomyces*.

In einer weiteren Ausführungsform betrifft die Erfindung die Verwendung von erfindungsgemäßen Vektoren, erfindungsgemäßen Expressionskassetten oder erfindungsgemäßen Wirtszellen, insbesondere der Gattung *Agrobacterium*, zur Transformation von Pflanzen, Pflanzenzellen, -geweben oder -teilen,.

In einer besonders bevorzugten Ausführungsform sind die erfindungsgemäßen Wirtszellen Pflanzenzellen, die im folgenden als "transgene Pflanzenzellen" bezeichnet werden.

Ferner betrifft die vorliegende Erfindung auch Pflanzen, die erfindungsgemäße Pflanzenzellen enthalten. Diese können grundsätzlich jeder beliebigen Pflanzenart, -gattung, -familie, -ordnung bzw. -klasse angehören, die gewerblich nutzbar sind. Es können sowohl monokotyle als auch dikotyle Pflanzen sein. Vorzugsweise sind die erfindungsgemäßen Pflanzen Nutzpflanzen, d.h. Pflanzen, die von agrarwirtschaftlichem, forstwirtschaftlichem und/oder gartenbauwirtschaftlichem Interesse sind. Bevorzugt sind dabei landwirtschaftliche Nutzpflanzen, insbesondere Getreidearten wie z.B. Weizen, Hafer, Gerste, Roggen, Mais, Reis, Futter- und Weidegräser (wie z.B. Alfalfa, weißer oder roter Klee), insbesondere Weizen.

In einer weiteren Ausführungsform betrifft die vorliegende Erfindung auch Verfahren zur Herstellung von transgenen Pflanzenzellen und Pflanzen, dadurch gekennzeichnet, daß man Pflanzenzellen, -gewebe oder -teile oder Protoplasten mit einem erfindungsgemäßen Nukleinsäuremolekül, einem erfindungsgemäßen Vektor, einer erfindungsgemäßen Expressionskassette oder gegebenenfalls mit einer erfindungsgemäßen Wirtszelle, vorzugsweise einem Mikroorganismus transformiert, die transformierten Zellen, Gewebe, Pflanzenteile oder Protoplasten in einem Wachstumsmedium kultiviert und im Fall der Herstellung transgener Pflanzen daraus Pflanzen regeneriert.

In einer weiteren Ausführungsform betrifft die Erfindung die Verwendung von erfindungsgemäßen Vektoren, Expressionskassetten oder gegebenenfalls Wirtszellen zur Herstellung transgener Wirtszellen, insbesondere transgener Pflanzenzellen und Pflanzen.

In einer weiteren Ausführungsform betrifft die Erfindung ein Verfahren zur karyopsenspezifischen Genexpression in Pflanzen, worin eines oder mehrere der erfindungsgemäßen Nukleinsäuremoleküle direkt oder mittels eines oder mehrerer der erfindungsgemäßen Vektoren, Expressionskassetten oder Wirtszellen stabil in das Genom einer Pflanzenzelle integriert wird und aus besagter Pflanzenzelle eine Pflanze regeneriert wird.

In einer weiteren Ausführungsform betrifft die Erfindung ein Verfahren zur karyopsenspezifischen Gensuppression in Pflanzen, worin eines oder mehrere der erfindungsgemäßen Nukleinsäuremoleküle direkt oder mittels eines oder mehrerer der erfindungsgemäßen Vektoren, Expressionskassetten oder Wirtszellen stabil in das Genom einer Pflanzenzelle integriert wird und aus besagter Pflanzenzelle eine Pflanze regeneriert wird, vorzugsweise mittels Cosuppression.

Die erfindungsgemäßen Pflanzen können nach Verfahren hergestellt werden, die dem Fachmann bekannt sind, z.B. durch Transformation pflanzlicher Zellen oder Gewebe und Regeneration ganzer Pflanzen aus den transformierten Zellen bzw. dem Gewebe.

Für die Einführung von DNA in eine pflanzliche Wirtszelle stehen grundsätzlich eine Vielzahl von molekularbiologischen Arbeitstechniken zur Verfügung. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, die Injektion, die Elektroporation von DNA, die Einbringung der DNA mittels des biolistischen Ansatzes etc..

Bei der Injektion, Elektroporation und Transformation mittels biolistischer Methode ('particle gun') von DNA in Pflanzenzellen werden an sich keine speziellen Anforderungen an die verwendeten Plasmide gestellt. Es können einfache Plasmide wie z. B. pUC-Derivate verwendet werden. Sollen aber aus derartig transformierten Zellen ganze Pflanzen regeneriert werden, ist z.B. die Anwesenheit eines selektierbaren Markergens notwendig.

Je nach Einführungsmethode gewünschter Gene in die Pflanzenzelle können weitere DNA-Sequenzen erforderlich sein. Werden z. B. für die Transformation der Pflanzenzelle das Ti- oder Ri- Plasmid verwendet, so muss mindestens die rechte Begrenzung, häufig jedoch die rechte und linke Begrenzung der Ti- und Ri- Plasmid T-DNA als Flankenbereich mit den einzuführenden Genen verbunden werden.

Werden für die Transformation Agrobakterien verwendet, muss die einzuführende DNA in spezielle Plasmide kloniert werden, und zwar entweder in einen intermediären Vektor oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält ausserdem die für den Transfer der T-DNA notwendige vir-Region. Intermediäre Vektoren können nicht in Agrobakterien replizieren. Mittels eines Helferplasmids kann der intermediäre Vektor auf Agrobacterium tumefaciens übertragen werden (Konjugation). Binäre Vektoren können sowohl in E.coli als auch in Agrobakterien replizieren. Sie enthalten ein Selektionsmarker-Gen und einen Linker oder Polylinker, welche von der rechten und linken T-DNA Grenzregion eingerahmt werden. Sie können direkt in die Agrobakterien transformiert werden (Holsters et al., Mol. Gen. Genet. 163 (1978), 181-187). Das als Wirtszelle dienende Agrobakterium soll ein Plasmid, das eine vir-Region trägt, enthalten. Die vir-Region ist für den Transfer der T-DNA in die Pflanzenzelle notwendig.
Zusätzliche T-DNA kann vorhanden sein. Das derartig transformierte Agrobakterium wird zur Transformation von Pflanzenzellen verwendet.

Die Verwendung von T-DNA für die Transformation von Pflanzenzellen ist intensiv untersucht und ausreichend in EP 120 516 beschrieben worden; Hoekema, In: The Binary Plant Vector System Offsetdrukkerij Kanters B.V., Alblasserdam (1985), Chapter V; Fraley et al., Crit. Rev. Plant. Sci., 4, 1-46 und An et al. EMBO J. 4 (1985), 277-287.

Für den Transfer der DNA in die Pflanzenzelle können Pflanzen-Explantate zweckmässigerweise mit Agrobacterium tumefaciens oder Agrobacterium rhizogenes cokultiviert werden. Aus dem infizierten Pflanzenmaterial (z. B. Blattstücke, Stengelsegmente, Wurzeln, aber auch Protoplasten oder Suspensions-kultivierte Pflanzenzellen) können dann in einem geeigneten Medium, welches Antibiotika oder Biozide zur Selektion transformierter Zellen enthalten kann, wieder ganze Pflanzen regeneriert werden. Die so erhaltenen Pflanzen können dann auf Anwesenheit der eingeführten DNA untersucht werden. Andere Möglichkeiten der Einführung fremder DNA unter Verwendung des biolistischen Verfahrens oder durch Protoplastentransformation sind bekannt (vgl. z. B. Willmitzer, L., 1993 Transgenic plants. In: Biotechnology, A Multi-Volume Comprehensive Treatise (H.J. Rehm, G. Reed, A. Pühler, P. Stadler, eds.), Vol. 2, 627-659, VCH Weinheim-New York-Basel-Cambridge).

Monokotyle Pflanzen werden inzwischen routinemäßig mittels des biolistischen Ansatzes und mittels Agrobakterien transformiert (Komari et al., (1998); Advances in cereal gene transfer; Current Opinion in Plant Biotechnology 1, S. 161 ff.; Bilang et al. (1999), Transformation of Cereals, Genetic Engineering, 12, S. 113-148 Hrsg.: JK Setlow, Kluwer Academic / Plenum Publisher, New York).
Weitere geeignete Methoden sind die elektrisch oder chemisch induzierte DNA-Aufnahme in Protoplasten, die Elektroporation von partiell permeabilisierten Zellen, die Makroinjektion von DNA in Blütenstände, die Mikroinjektion von DNA in Mikrosporen und Pro-Embryonen, die DNA-Aufnahme durch keimenden Pollen und die DNA-Aufnahme in Embryon durch Quellung (zur Übersicht: Potrykus, Physiol. Plant (1990), 269-273).

Ferner betrifft die vorliegende Erfindung auch Vermehrungsmaterial und Emtegut von erfindungsgemäßen Pflanzen, die erfindungsgemäße Pflanzenzellen enthalten.

Der Begriff "Vermehrungsmaterial" umfaßt im Sinn der vorliegenden Erfindung jene Bestandteile der Pflanze, die zur Erzeugung von Nachkommen auf vegetativem oder generativem Weg geeignet sind. Für die vegetative Vermehrung eignen sich beispielsweise Stecklinge, Kalluskulturen, Rhizome, Wurzelstöcke oder Knolien, Anderes Vermehrungsmaterial umfaßt beispielsweise Früchte, Samen, Sämlinge, Protoplasten, Zellkulturen etc. Vorzugsweise handelt es sich bei dem Vermehrungsmaterial um Knollen oder Samen.

Weiterhin betrifft die vorliegende Erfindung die Verwendung der erfindungsgemäßen Promotoren oder der mittels des erfindungsgemäßen Verfahrens identifizierten Promotoren zur karyopsenspezifischen Expression von Transgenen in Pflanzenzellen oder Pflanzen.

Außerdem betrifft die vorliegende Erfindung die Verwendung der erfindungsgemäßen Promotoren oder der mittels des erfindungsgemäßen Verfahrens identifizierten Promotoren zur karyopsenspezifischen Cosuppression von Genen oder Transgenen in Pflanzenzellen oder Pflanzen.

Der Begriff "Transgen" bedeutet dabei eine in eine Pflanze künstlich eingeführte DNA-Sequenz, die ein oder mehrere der erfindungsgemäßen Nukleinsäuremoleküle enthalten.

Diese und andere Ausführungsformen sind dem Fachmann durch die Beschreibung und die Beispiele der vorliegenden Erfindung offenbart. Weiterführende Literatur kann zu einer der oben angeführten Methoden, Mitteln und Anwendungen, die im Sinne der vorliegenden Erfindung benötigt werden, sind dem Fachmann aus dem Stand der Technik bekannt. Zu diesem Zweck bieten sich unter anderem öffentliche Datenbanken an (z.B. "Medline"), die ggf. über Internet zur Verfügung stehen, z. B. unter der Adresse http://www.ncbi.nlm.nih.gov/PubMed/medline.html. Weitere Datenbanken und Adressen sind dem Fachmann geläufig und können ggf. dem Intemet entnommen werden, z. B. unter der Adresse http://www.lycos.com. Eine Übersicht über Quellen und Informationen zu Patenten bzw. Patentanmeldungen in der Biotechnologie ist in Berks, TIBTECH 12 (1994), 352-364 gegeben.
Zur detaillierteren Beschreibung der Erfindung wird einer der erfindungsgemäßen Promotoren durch SEQ ID No.1 dargestellt, bestehend aus 4683 Basen der genomischen Sequenz des isolierten ssll-Subklons p15/G wie durch DSM 14224 hinterlegt.

SEQ ID No.1 repräsentiert die DNA-Sequenz des genomischen ssll-Subklons p15/G (Größe des Inserts: 5057 Basenpaare). Die Nukleotide 1-4683 von Seq. ID No. 1 entsprechen dem 5'-flankierenden Bereich des Gens, d.h. dem ssll Promotor. Der Subklon p15/G enthält außerdem 374 Basen des Strukturgens der SSII (Position 4684 bis 5057). Ein Intron mit einer Länge von 91 Basen liegt an Position 4948 bis 5038.

Der isolierte cDNA-Klon der ssll (Walter (2000), Dissertation Uni Hamburg, Fachbereich Biologie, WO 97/45545 bzw. EMBL Datenbank U66377) zeigt mit der in SEQ ID No.1 aufgeführten genomischen Sequenz im 5'-untranslatierten Bereich (Position bis 4512 bis 4683) eine Homologie von 83,7%. Im ersten Exon (Position 4684-4947) besteht zu dieser cDNA-Sequenz eine 92,6%ige Sequenzidentität. Der 5'-untranslatierte Bereich und das erste Exon der unter SEQ ID No.1 aufgeführten Sequenz (Position 4596-4947) ist mit den ersten 352bp eines von Li *et al.* (Theor. Appl. Genet., (1999), 98: 1208-1216) publizierten cDNA-Klons einer ssll, WSSIIA und den ersten 318bp des von Gao *et al.* (2000, loc.cit.) publizierten cDNA-Klon wss2a-2 (AJ269503) identisch.

Der 5'-flankierende DNA-Bereich des isolierten genomischen Klons (SEQ ID. No.1, d.h. die Nukleotide 1-4683 von Seq. ID No. 1) wurde mittels Datenbankrecherchen mit bereits veröffentlichten Sequenzen verglichen. Es handelt sich bei dem Promotorbereich eines ssll-Gens aus Weizen um eine bisher unbekannte Sequenz.

Außerdem wurde der 5'-flankierend zum Startcodon gelegenen (SEQ ID No.1, Die Nukleotide 1-4683) beschriebenen DNA-Sequenz in der PLACE-Datenbank (http://www.dna.affrc.go.jp/htdocs/PLACE/; Web Signal Scan Program) nach DNA-Motiven mit Sequenzhomologie zu bekannten *cis*-regulatorischen Elementen durchsucht. In dem ssll-Promotor (d.h. Die Nukleotide 1-4683 der Seq. ID No. 1, wie durch DSM 14224 hinterlegt) wurden folgende Endosperm- bzw. samenspezifische *cis*-regulatorische DNA-Elemente identifiziert:

| **Name (Gen; Organismus) Signalsequenz** | **Position (Strang)** | |
|---|---|---|
| -300 Element (Gliadine, Glutenine; | Position 941 (+) | TGHAAARK |
| *T. aestivum*) | Position 3134 (+) | TGHAAARK |
| | Position 3406 (+) | TGHAAARK |

| **Position 580 (-) TGHAAARK** | | |
|---|---|---|
| -300 Motiv (Zein; *Z. mais)* | Position 4467 (+) | TGTAAAG |
| Napin-Motiv (2S Albumin; *B. napus*) | Position 3311 (-) | TACACAT |
| (CA)ₙ Element (napA; *B. napus)* | Position 2285 (+) | CNAACAC |

| | | |
|---|---|---|
| | **Position 2714 (-)** | **CNAACAC** |
| | Position 4420 (+) | CNAACAC |
| ACGT Box (Glu-B1, *O. sativa*) | Position 2193 (+) | GTACGTG |
| | Position 2477 (+) | GTACGTG |
| Amylase-Box (α-Amylase, *T. aestivum*) | Position 3534 (+) | TAACARA |
| CGACG-Element (Amylase, *O. sativa*) | Position 195 (+) | CGACG |
| | Position 957 (+) | CGACG |

| **Position 1181 (+) CGACG** | | |
|---|---|---|
| | Position 2321 (+) | CGACG |
| | Position 1331 (-) | CGACG |
| | Position 2015 (-) | CGACG |
| | Position 2277 (-) | CGACG |
| | Position 4481 (-) | CGACG |
| E-Box (napA; *B. napus*) (= G-Box) | Position 72 (+) | CACGTG |
| | Position 866 (+) | CANNTG |
| | Position 1568 (+) | CANNTG |
| | Position 1594 (+) | CANNTG |
| | Position 2603 (+) | CANNTG |
| | Position 2980 (+) | CANNTG |
| | Position 3290 (+) | CANNTG |
| | Position 3447 (+) | CANNTG |
| | Position 3751 (+) | CANNTG |
| | Position 4032 (+) | CANNTG |
| | Position 4110 (+) | CANNTG |
| | Position 4174 (+) | CANNTG |
| | Position 4295 (+) | CACGTG (= G-Box) |
| RY repeat (Gy2; *V. faba*) | Position 2353 (+) | CATGCATG |

| **Position 2353 (+) CATGCAT** | | |
|---|---|---|
| | Position 2532 (+) | CATGCAT |
| | Position 2354 (-) | CATGCAT |
| | Position 2471 (-) | CATGCAT |
| | Position 4146 (+) | CATGCAT |
| RY repeat (Legumin; *G*. *max*) | Position 2088 (+) | CATGCAY |
| RY repeat (napA; *B. napus)* | Position 733 (-) | CATGCA |
| | Position 2355 (-) | CATGCA |
| | Position 2472 (-) | CATGCA |
| | Position 2601 (-) | CATGCA |
| SEF1 Motiv (7S Globulin; *G. max*) | Position 2871 (+) | ATATTTAWW |
| | Position 3108 (+) | ATATTTAWW |
| | Position 2938 (-) | ATATTTAWW |
| SEF3 Motiv (7S Globulin; *G. max*) | Position 403 (+) | AACCCA |
| | Position 1114 (-) | AACCCA |
| SEF4 Motiv (7S Globulin; *G. max*) | Position 845 (+) | RTTTTTR |
| | Position 2787 (+) | RTTTTTR |
| | Position 838 (-) | RTTTTTR |
| | Position 3047 (-) | RTTTTTR |
| | Position 3156 (-) | RTTTTTR |
| | Position 3274 (-) | RTTTTTR |
| | Position 3785 (-) | RTTTTTR |
| | Position 2876 (-) | RTTTTTR |
| | Position 3049 (-) | RTTTTTR |
| | Position 3099 (-) | RTTTTTR |
| | Position 3113 (-) | RTTTTTR |
| | Position 3198 (-) | RTTTTTR |
| | Position 3306 (-) | RTTTTTR |
| | Position 3571 (-) | RTTTTTR |
| | Position 3681 (-) | RTTTTTR |

Sequenzhomologien zu Elementen, die an einer durch Zucker regulierten Genexpression beteiligt sind wurden an folgenden Positionen gefunden:

| **Name (Gen; Organismus) Signalsequenz** | **Position (Strang)** | |
|---|---|---|
| ACGTA-Box (α-Amylase; *O. sativa*) | Position 4450(+) | TACGTA |
| CGACG-Element (AMY3; *O. sativa*) | Position 195 (+) | CGACG |
| | Position 957 (+) | CGACG |
| | Position 1181 (+) | CGACG |
| | Position 2321 (+) | CGACG |
| | Position 1331 (-) | CGACG |
| | Position 2015 (-) | CGACG |
| | Position 2277 (-) | CGACG |
| | Position 4481 (-) | CGACG |
| | Position 4484 (-) | CGACG |
| | Position 4688 (-) | CGACG |
| SURE1 (Sbe2.2; *A. thaliana*) | Position 3979 (+) | AACAGAAAA |

Sequenzhomologien zu DNA-Elementen, die an einer hormonell regulierten Genexpression durch ABA oder GA beteiligt sind, wurden an folgenden Positionen gefunden:

| Name (Gen; Organismus) Signalsequenz | Position (Strang) | |
|---|---|---|
| ABRE (rd22, *A. thaliana*) | Position 71 (+) | RYACGTGGY |
| ABRE Motiv A (Osem; *O.sativa*) | Position 2478 (+) | TACGTGTC |
| ABRE (Em, *T. aestivum*) | Position 1219 (+) | ACGTSSSC |
| | Position 1179 (-) | ACGTSSSC |
| | Position 2044 (-) | ACGTSSSC |
| EMBP1 (Em, T. aestivum) | Position 72 (+) | CACGTGGC |
| Pyrimidin-Box (EBP-1; *H. vulgare*) | Position 3459 (-) | TTTTTTCC |
| | Position 3479 (-) | TTTTTTCC |
| | Position 3608 (-) | TTTTTTCC |
| DPBF Motiv (Dc3; *D.carota*) | Position 4463 (-) | ACACNNG |
| LTRE (cor15a; *A.thaliana*) | Position 956 (+) | CCGAC |
| | Position 1772 (+) | CCGAC |
| | Position 2143 (+) | CCGAC |
| | Position 2222 (+) | CCGAC |
| | Position 2320 (+) | CCGAC |
| | Position 87 (-) | CCGAC |
| | Position 397 (-) | CCGAC |
| | Position 780 (-) | CCGAC |
| | Position 1214 (-) | CCGAC |
| | Position 4689 (-) | CCGAC |

Sequenzhomologien zu Elementen, die an einer hormonell regulierten Genexpression durch Auxin bzw. Ethylen beteiligt sind, wurden an folgenden Positionen gefunden:

| Name (Gen; Organismus) Signalsequenz | Position (Strang) | |
|---|---|---|
| ASF-1 Motiv (35S; CaMV) | Position 192 (+) | TGACG |
| | Position 775 (+) | TGACG |
| | Position 819 (+) | TGACG |
| | Position 2150 (+) | TGACG |
| | Position 3694 (+) | TGACG |
| | Position 16 (-) | TGACG |
| | Position 1406 (-) | TGACG |
| | Position 1803 (-) | TGACG |
| | Position 4029 (-) | TGACG |
| | Position 4046 (-) | TGACG |
| Auxin response f. (ARF; *A.thaliana*) | Position 1539 (-) | TGTCTC |
| NtBBF1 Motiv (rolB; *A. rhizogenes*) | Position 3702 (+) | ACTTTA |
| Ethylen RE (E4; *L.esculentum*) | Position 3270 (+) | AWTTCAAA |

Sequenzhomologien zu DNA-Elementen, die für eine Licht- oder Temperatur-regulierte Genexpression stehen, wurden an folgenden Positionen gefunden:

| Name (Gen; Organismus) Signalsequenz | Position (Strang) | |
|---|---|---|
| I-Box (Monokotyle und Dikotyle) | Position 2883 (+) | GATAA |
| | Position 463 (-) | GATAA |
| | Position 2907 (-) | GATAA |
| | Position 3065 (-) | GATAA |
| | Position 3804 (-) | GATAA |
| | Position 3920 (-) | GATAA |
| | Position 3920 (-) | GATAA |
| | Position 4087 (-) | GATAA |
| LTRE-1 (blt4.9; *H. vulgare*) | Position 179 (-) | CCGAAA |
| LTRE (Iti; *A. thaliana*) | Position 2221 (+) | ACCGACA |
| LTRE (cor15a; *A.thaliana*) | Position 956 (+) | CCGAC |
| | Position 1772 (+) | CCGAC |
| | Position 2143 (+) | CCGAC |
| | Position 2222 (+) | CCGAC |
| | Position 2320 (+) | CCGAC |
| | Position 4689 (+) | CCGAC |
| | Position 87 (-) | CCGAC |
| | Position 397 (-) | CCGAC |
| | Position 780 (-) | CCGAC |
| | Position 1214 (-) | CCGAC |

Neben den beschriebenen Sequenzmotiven wurden in dem Promotor Homologien zu DNA-Motiven für allgemeine Transkriptionsfaktoren (z.B. GT1-Consensus, G-Boxen, DOF-Boxen, GATA-Motive, Myb- und Myc-Boxen; zur Information s. PLACE Datenbank), sowie T-Boxen und ARS-Elemente (Gasser S.M. *et al.* (1989) Intnatl Rev Cyto 119:57-96) gefunden.

Der unter SEQ ID No.1 aufgeführte ssll-Promotor weist außerdem bisher nicht beschriebene Sequenzmotive auf. Dazu gehört ein Motiv der Sequenz 5'-AAAAATGT-3', das insgesamt neun mal im Bereich von 3009 bis 3329 der unter SEQ ID No.1 aufgeführten Sequenz auftritt (Positionen: 3009, 3030, 3114, 3157, 3177, 3199, 3275, 3307, 3321). Im Gegensatz zu diesem Motiv hat das -300 Element, auch Prolamin-Box; genannt, das Sequenzmotiv 5'-TGTAAAG-3' und ist etwa 300 Nukleotide vom Startpunkt der Transkription in Promotoren von Hordeinen (Gerste), Gliadinen und LMW-Gluteninen (Weizen), sowie α-Zeinen (Mais) lokalisiert (Forde *et al.* (1985) Nucleic Acid Research 13: 7327-7339; Mena *et al.* (1998) Plant J. 16: 53-62).

Sich direkt wiederholende, kurze Sequenzmotive befinden sich an Position 4221 (TCTA)₄, an Position 2304 (GCCT)₃ und Position 2364 (GCT)₃. Ein direktes Repeat der Sequenz AAAAATGTAATCAAGCATTT befindet sich an den Positionen 3199 und 3275. Im 5'-untranslatierten Bereich, direkt vor dem Translationsstart (Position 4671 in SEQ ID No.1), befindet sich eine GC-reiche Sequenz (CCCGGCCGCC), die auch im 5'-untranslatierten Bereich des Mais zSSlla cDNA-Klons vor dem Translationsstart vorliegt (Genbank Acc.No. AF019296; Ham *et al*. (1998) Plant Mol. Biol. 37: 639-649).

Der in der deutschen Patentanmeldung DE10032379.0 offenbarte und durch DSM 13397 hinterlegte genomische ssll-Subklon p8/C stellt ein Fragment der Sequenz ID No. 1 dar. Insofern ist der Inhalt der DE10032379.0 ausdrücklich durch Referenz Teil dieser Patentanmeldung.

### Hinterlegung von Mikroorganismen:

Das erfindungsgemäße Nukleinsäuremolekül SEQ ID No. 1 wurde bei der Deutschen Sammlung für Mikroorganismen und Zellkulturen (DSMZ) in Braunschweig, Deutschland gemäß Budapester Vertrag mittels Plasmid DNA hinterlegt:
Am 06. April 2001 erfolgte die Hinterlegung von Plasmid p15/G enthaltend SEQ ID No. 1 unter der Hinterlegungsnummer DSM 14224 bei der DSMZ.

### Klonierungsverfahren

Zur Klonierung in *E.coli*-Bakterienstämme wurden die Vektoren pBlueskript™II SK(+/-) bzw. KS(+/-) Phagemid Vektoren (Stratagene GmbH, Heidelberg, Deutschland) und Lambda Fix®II/XhoI Klonierungsvektor (Stratagene GmbH, Heidelberg, Deutschland) verwendet.

### Bakterienstämme

Für die Blueskript-Vektoren wurden die *E.coli*-Stämme DH5α (Life Technologies, Eggenstein, Deutschland) und Epicurian Coli SURE® (Stratagene GmbH, Heidelberg, Deutschland) verwendet. Für die Bakteriophagen-Vektoren wurde der Epicurian Coli Stamm XL1-Blue MRA (Stratagene) verwendet.

Für grundlegende molekularbiologische Arbeitstechniken oder beispielsweise Pufferzusammensetzungen sei auf Sambrook et al. ((1989), Molecular Cloning, A Laboratory Manual, Second Edition, Cold Spring Harbour Laboratory Press) verwiesen.

Die nachfolgenden Beispiele erläutern die Erfindung ohne sie in irgendeiner Hinsicht zu beschränken.

### Ausführungsbeispiele

### 1. Herstellung der genomischen Weizenbank

Zur Herstellung der genomischen Weizenbanken wurde Gesamt-DNA aus etiolierten Keimlingen von Triticum aestivum L. cv. "Florida" isoliert. Zur Anzucht steriler etiolierter Keimlinge wurden reife Karyopsen für 20 min mit 1% NaOCl, 0,1% (v/v) Mucasol® (Merz & Co., Frankfurt, Deutschland) inkubiert und anschließend 3x mit Aqua bidest gewaschen. Die Karyopsen wurden auf sterilem MS-Medium (Murashige & Skoog (1962), Physiol. Plant. 15: 473-479) ausgelegt, dem 0,3% (w/v) GELRITE® (Carl Roth GmbH & Co., Karlsruhe, Deutschland) zur Verfestigung zugesetzt wurde. Das Wachstum erfolgte bei 26°C in Dunkelheit. Vierzehn Tage nach dem Plattieren wurden die Keimlinge abgeschnitten und in flüssigem Stickstoff eingefroren.

Der partielle Verdau der genomischen DNA erfolgte mit den Restriktionsenzymen BamH I bzw. Sau3A 1 (Life Technologies, Eggenstein, Deutschland). Hierzu wurden 3 Aliquots von jeweils 100 µg genomischer DNA mit 150 µl des entsprechenden Restriktionspuffers in einem Gesamtvolumen von 1,5 ml und 12,5 Units, 6,25 Units bzw. 3,125 Units des Restriktionsenzyms BamH 1 bzw. 1,56 Units, 0,78 Units bzw. 0,39 Units Sau3A 1 für 1 h bei 37°C restringiert. Aliquots der partiell restringierten DNA wurden anschließend gelelektrophoretisch auf den Restriktionsgrad analysiert. Die Restriktionsenzyme wurden durch einmalige Phenol/Chloroform/ Isoamylalkohol-Extraktion (25:24:1, v/v) und Chloroform/Isoamylalkohol-Extraktion (24:1, v/v) aus den Ansätzen entfernt. Anschließend wurde Saccharose bis zu einer Endkonzentration von 10% (w/v) zu jedem Ansatz zugegeben.

Die Größenfraktionierung der partiell restringierten DNA erfolgte in kontinuierlichen Saccharose-Gradienten (10-40% w/v) (Sambrook *et al.* (1989)). Die Aliquots der partiell restringierten DNA-Moleküle wurden vor dem Beladen jeweils eines 15 ml Saccharosegradienten für 10 min auf 68°C erwärmt und dann auf 20°C abgekühlt. Die Zentrifugation der Gradienten erfolgte für 24 h, bei 20°C und 22000 rpm (Beckmann, Rotor SW 40). Nach der Zentrifugation wurden die einzelnen Zentrifugenröhrchen im Boden angestochen und jeweils 500 µl Aliquots gesammelt. Von den einzelnen Fraktionen wurden 30 µl in einem 0,5%igen Agarosegel aufgetrennt und die Größenverteilung der DNA in den einzelnen Fraktionen bestimmt. Fraktionen, die genomische DNA von ca. 4,0 kb und größer enthielten, wurden vereinigt. Die Saccharose aus den Proben wurde durch Dialyse gegen Tris/EDTA-Puffer (10 mM/1mM) entfernt. Anschließend wurden die Proben mit 2-Butanol eingeengt und die DNA aus den Proben mit 2 Volumenteilen EtOH (99,8%)/ 2 M Ammoniumacetat (Endkonzentration) bei Raumtemperatur (RT) ausgefällt.

Zum Auffüllen der 3'-Enden der partiell restringierten DNA wurden 20 µg der BamH I bzw. Sau3A I restringierten DNA mit 1 mM dATP, 1 mM dGTP (Roche, Mannheim), 6 µl 10x Pfu Reaktionspuffer und 10 Units native Pfu-DNA Polymerase (DNA-Polymerase mit "proof-reading" Aktivität; Stratagene GmbH, Heidelberg, Deutschland) in einem Endvolumen von 60 µl inkubiert. Die Reaktion wurde bei 72°C für 1 h 30 min durchgeführt. Anschließend erfolgte eine Phenol/Chloroform/Isoamylalkohol-, sowie eine Chloroform/Isoamylalkohol-Extraktion der DNA und nachfolgend eine Präzipitation der DNA mit 1/10 Vol. 3M NaAc und 2,5 Volumenteilen EtOH (absolut).

### 1.1. Ligation in Lambda Fix® II/Xho I Partial Fill-In Vektoren (Stratagene GmbH, Heidelberg, Deutschland)

Die BamH I bzw. Sau3A I restringierte genomische DNA wurde in den Lambda Fix® II/Xho I Klonierungsvektor nach Angaben des Herstellers (Stratagene GmbH, Heidelberg, Deutschland) ligiert. Der Ligationsansatz enthielt: 1 µl des Lambda Fix® II Vektors, 0,4 µg BamH I bzw. Sau3A I restringierte genomische DNA, 0,5 µl 10x Ligationspuffer, 2 Weiss Units T4 DNA-Ligase (MBI Fermentas GmbH, St. Leon-Rot, Deutschland); Weiss et al. (1968) J. Biol. Chem., 243:4543-4555) in einem Endvolumen von 5 µl.

### 1.2. In vitro Verpackung der Ligationsprodukte

Zur Verpackung der Lambda Phagen wurde das *in vitro*-Verpackungskit "Gigapack® II Gold" der Firma Stratagene (Stratagene GmbH, Heidelberg, Deutschland) verwendet und den Angaben des Herstellers gefolgt. Von den Ligationsansätzen wurde jeweils 1 µl zu den Verpackungsansätzen dazugegeben und im Folgenden den Angaben des Herstellers gefolgt.

### 1.3. Anzucht der Bakterien zur Phagenvermehrung

Zur Phagenvermehrung wurde der *E.coli*-Bakterienstamm XL1-Blue MRA (P2) verwendet. Die Bakterien wurden in LB-Medium mit 10 mM MgSO₄, 0,2% (w/v) Maltose bis zu einer OD₆₀₀ = 0,5 bei 37°C, 180 rpm angezogen. Anschließend wurden die Bakterien bei 2000 rpm, 10 min, 4°C pellettiert und der Überstand verworfen. Das Bakterienpellet wurde in 10 mM MgSO₄ resuspendiert und die Bakteriendichte auf OD₆₀₀ = 0,5 eingestellt.

Zur Phagenvermehrung wurden aus den Verpackungsansätzen 1µl aus den Originalansätzen bzw. 1:10 Verdünnung der Originalansätze mit 200µl Bakteriensuspension (OD₆₀₀ = 0,5) gemischt und 15 min bei 37°C inkubiert. Anschließend wurden die einzelnen Ansätze mit 3 ml TOP-Agarose (48°C) gemischt und auf NZY-Festmedium nach Herstellerangaben (s.o. Lambda Fix® II/Xho I Partial Fill-In Vektoren, Stratagene) plattiert. Die Platten wurden für ca. 16 h bei 33°C inkubiert.

Die Phagentiter der Sau3A I bzw. der BamH I genomischen Banken wurden durch Auszählen der Phagenplaques bestimmt. Für die Sau3a I bzw. die BamHI Primärbanken wurden Phagentiter von 2,2 × 10⁷ pfu/ml bzw. 1,4 × 10⁷ pfu/ml ermittelt. Zur Bestimmung der durchschnittlichen Insertgrößen wurden von jeder Bank 10 einzelne Phagenklone amplifiziert, die Phagen-DNA isoliert (Sambrook *et al*., 1989) und die Insertgrößen nach Restriktionsverdau und gelelektrophoretischer Auftrennung ermittelt. Die durchschnittliche Insertgröße beträgt ca. 15,0 Kb für die BamH I bzw. 15,6 Kb für die Sau3A I Bank.

### 1.4. Amplifizierung der genomischen Banken

Zur Herstellung repräsentativer, amplifizierter genomischer Banken wurden von jeder Bank ca. 4,5 Millionen pfu plattiert. Die Ampflifizierung erfolgte nach den Angaben des Herstellers (Stratagene). Die Phagentiter der amplifizierten Banken betrug 6,3 × 10⁹ pfu/ml (BamHI-Bank) bzw. 2,0 × 10⁹ pfu/ml (Sau3A I-Bank).

### 2. Durchmustern der genomischen Banken

Die Identifizierung und Isolierung von Phagenklonen, deren genomische Inserts Sequenzen der ssll-Gene tragen, erfolgte über Kolonie-Plaque-Hybridisierung. Zum Durchmustern der genomischen Banken wurden ca. 500.000 Phagen von jeder Bank ausplattiert. Das Ausplattieren der Phagen und Abziehen der Platten erfolgte nach Standardprotokollen (Sambrook *et al*., 1989; Stratagene Lambda Fix® II Manual). Als genspezifische Sonde wurde ein 709 bp großes DNA-Fragment eines cDNA-Klones einer ssll (WO 97/45545 A1, EMBL Datenbank U66377, Walter (2000), Dissertation Uni Hamburg, Fachbereich Biologie, Position 1264-1973) eingesetzt.

Die ssll-Sonde wurde mit sequenzspezifischen Primem über eine PCR-Reaktion aus einem isolierten ssll cDNA-Klon amplifiziert. Die Markierung des 709 bp großen Amplifikationsprodukts (Position 1264-1972 der ssll cDNA) erfolgte während der PCR-Reaktion durch den Einbau von DIG-dUTPs (Roche Diagnostics GmbH, Mannheim).

Der PCR-Reaktionsansatz setzte sich folgendermaßen zusammen:
10µl PCR-Puffer (10-fach, ohne Mg; Life Technologies)
3µl MgCl₂ (50mM; Life Technologies)
7 µl DIG dUTPs (1nmol/µl; Roche Diagnostics GmbH, Mannheim)
je 8µl dATP, dCTP, und dGTP (je 2,5mM)
5µl dTTP (2,5mM)
5µl Primer LW2 (10pmol/µl)
5µl Primer LW9 (10pmol/µl)
10ng Template (cDNA-Klon der ssll)
0,5µl Taq-Polymerase (5U/µl; Life Technologies)
ad 100µl H₂O bidest

Die Bedingungen für die PCR waren folgendermaßen:
96°C, 5min
96°C, 1 min
58°C, 1 min
72°C, 1min (IV.→ II. 29 Schleifen)
72°C, 5min

Die Sequenzen der ssll-spezifischen Primer für die Amplifikation der PCR-Sonde waren:

LW2: 5´-CTGCTGGACAGGATATGGAA- 3´ (SEQ ID No. 11)

LW9: 5'-TCGCGCTGCAGGGCCTCCTT- 3' (SEQ ID No. 12)

Die Prähybridisierung der Filter erfolgte in 5x SSC, 3% Blockingreagenz (Boehringer Mannheim), 0,2% Na-dodecylsulfat (SDS), 0,1% N-Laurylsarcosin und 30µg/ml Heringssperma-DNA bei 65°C im Wasserbad. Die Hybridisierung mit den DIG-markierten DNA-Sonden (6ng/ml Hybridisienrngslösung) erfolgte über Nacht bei 65°C im beschriebenen Standard-Hybridisierungspuffer.
Alle weiteren Schritte der Chemilumineszenz-Reaktion mit CSPD® erfolgten nach Angaben des Herstellers (Roche Diagnostics GmbH, Mannheim, Deutschland).

Positive Plaques wurden ausgestochen und über zwei weitere Runden der Amplifikation und Plaque-Filterhybridisierung vereinzelt. Die DNA der isolierten positiven Phagen wurde mittels des Qiagen® Lambda Kit (Qiagen GmbH, Hilden, Deutschland) gereinigt, mit verschiedenen Restriktionsenzymen geschnitten und nach Agarose-Gelelektrophorese in Southern-Hybridisierungen mit der oben beschriebenen Sonde analysiert.

### 3. Subklonierung der λ-Phagenklone in bakterielle Vektoren (pBlueskript™ II)

Positive Phagenklone der genomischen Bank wurden mit der oben genannten genspezifischen Sonde (709 bp) identifiziert. Die genomischen Inserts der positiven Phagenklone wurden mit verschiedenen Restriktionsenzymen bzw. Enzymkombinationen in kürzere Fragmente geschnitten. Die resultierenden Subfragmente wurden in bakterielle Vektoren (pBlueskript™ II SK(+/-) bzw. KS(+/-) Phagemid Vektoren; Stratagene GmbH, Heidelberg, Deutschland) kloniert.

Über Southem-Hybridisierungen erfolgte die Isolierung von ssll-spezifischen Subklonen mit 5'-stromaufwärts gelegenen regulativen Elementen. Dazu wurde eine weitere Digoxigenin-markierte Sonde hergestellt, die im äußersten 5'-Bereich der ssll cDNA-Sequenz liegt. Die Sonde erstreckt sich vom 5'-untranslatierten Bereich des cDNA-Klons der ssll bis in das erste Exon (Position 1-218 der ssll-cDNA aus WO 97/45545 A1). Das Fragment wurde aus der cDNA der ssll (in pBlueskript™ SK II) über einen Restriktionsverdau mit Smal herausgeschnitten und nach der Auftrennung über eine Agarosegelelektrophorese isoliert. Die Markierung des Smal-Fragmentes erfolgte über ,Random Priming' mit dem DIG DNA Labeling Kit nach den Angaben des Herstellers (Roche Diagnostics GmbH, Mannheim, Deutschland).

Nach einem Verdau des Phagenklons 15 mit dem Enzym Xbal wurde ein Subklon mit 4682 Basenpaaren 5'- flankierend seines Strukturgens identifiziert (p15/G; SEQ ID No.1, wie am 6.4.2001 bei der DSZM in Braunschweig unter der Nummer DSM 14224 hinterlegt). Der Verdau mit SacI resultierte in einem Subklon mit 2462 Basenpaaren, 5'- flankierend des Strukturgens gelegen, der dem Klon p8/C entspricht, hinterlegt bei der DSZM in Braunschweig, Deutschland unter der Nummer DSM 13397 (entsprechend SEQ ID No.2 aus der deutschen Patentanmeldung DE 10032379.0). Der Subklon 15/G wurde vollständig sequenziert und zur Klonierung von Promotortestvektoren verwendet.

### 4. Sequenzanalysen

Zur Sequenzierung der genomischen Klone der ssll wurde die Firma SeqLab GmbH (Göttingen, Deutschland) beauftragt.

### 5. Klonierungen von Promotor-Testvektoren

Die Funktionsfähigkeit des in SEQ ID No.1 angeführten 5'-flankierenden DNA-Bereichs wurde in transienten und stabilen Expressionsanalysen überprüft. Als Reportergen wurde das Gen der β-Glucuronidase (GUS) verwendet (Jefferson (1987) Plant Molecular Biology Reporter 5(4): 387-405). Es wurden Promotortestvektoren kloniert, in denen die kodierende Region des gus-Gens (uidA) unter der Kontrolle des in SEQ ID No.1 (Die Nukleotide 1-4683) aufgeführten 5'-flankierenden DNA-Bereichs steht. Die Klonierung erfolgte als transkriptionale Fusion. Zunächst wurde das uidA-Gen über einen partiellen Verdau zusammen mit dem nos-Terminator aus dem Vektor pCal-GUS (uidA-Gen unter Kontrolle des CaMV 35S-Promotors; Chris Warren, Stanford Universität, unveröffentlicht) herausgeschnitten und hinter die Multiple Cloning Site von pBluescript (Stratagene) kloniert. Der so erzeugte promotorlose Vektor (uidA-nos) wurde für die weiteren Klonierungen verwendet.

Auch die 5'-untranslatierte Leadersequenz einer mRNA kann einen Einfluß auf die gewebespezifische Expression eines Gens haben (Rouster *et al*.(1998) Plant J. 15 (3): 435-40). Die klonierten Promotortestvektoren beinhalten daher diesen Bereich des ssll -Gens. In der gewählten Klonierungsstrategie liegt das Translationsstart-Codon der ß-Glucuronidase an der Position des Start-Codons des ssll-Gens.

### 5.1. Klonierung der ssll-Promotortestvektoren

Die Klonierung eines ssll-Promotortestvektors durch transkriptionale Fusion des ssll-Promotors mit dem Reportergen uidA wurde mit der Methode des "Splicing by Overlap Extension" (Horton (1997) Methods in Molecular Biology Vol.67: PCR Cloning Protocols (14): 141149, White Humana Press Inc.) durchgeführt.

Zunächst wurde dazu ein 2499bp großes Fragment des genomischem ssll-Subklons p15/G über einen Restriktionsverdau mit Sacl (Position 2241) und Smal (Position 4740) in das promotorlose Plasmid uidA-nos kloniert.

Die Erzeugung von Intermediär-Produkten für die Methode des "Splicing by Overlap Extension" erfolgte unter Einsatz folgender Primerpaare (analog DE 10032379.0):
a) Amplifizierungsreaktion mit genomischem ssll-Subklon als Matrize: SOE-A 5´-TCACGTGGATTCTGCAACCTC -3´ (SEQ ID No. 13) 
SOE-B 5´-CAGGACGGACCATGGCGGCGGCCGGGAT -3´ (SEQ ID
No. 14)
b) Amplifizierungsreaktion mit Plasmid pCalGUS als Matrize:

SOE-C 5´-CGCCGCCATGGTCCGTCCTGTAGAAACCC -3´ (SEQ
ID No. 15)

SOE-D 5′-GTGATGTCAGCGTTGAACTGC -3′ (SEQ ID No. 16)

Die Reaktionen erfolgten nach Horton (Methods in Molecular Biology (1997) Vol.67: PCR Cloning Protocols (14): 141149, White Humana Press Inc.), die Bedingungen für die PCR waren:
I. 94°C, 90sec
II. 94°C, 1min
III. 64°C, 1min
IV. 72°C, 1min (IV. → II. 20 Schleifen)
V. 72°C, 3min

Die Amplifizierung des PCR-Produkts für die Klonierung erfolgte mit den Primersequenzen SOE-A und SOE-D. Als Matrize dienten die erzeugten Intermediärprodukte. Der Reaktionsansatz erfolgte nach Horton (Methods in Molecular Biology (1997) Vol.67: PCR Cloning Protocols (14): 141149, White Humana Press Inc.), die PCR-Reaktionsbedingungen waren:
I. 96°C, 2min
II. 94°C, 1min
III. 68°C, 2min
IV. 72°C, 2min (IV. → II. 25 Schleifen)
V. 72°C, 10min

Die Klonierung des resultierenden PCR-Produktes zwischen dem ssll-Promotor und dem uidA-Gen erfolgte nach einem Restriktionsverdau mit den Enzymen Not I (Schnittstelle im ssll-Promotor, Position 4402) und Bal I (Schnittstelle im uidA-Gen).
Das so entstandene ssll-Promotortestkonstrukt trägt 2443bp 5'-flankierend des ssll-Gens (-2,45 ssll/GUS). Durch die Ligation der fehlenden distalen 2240bp des ssll-Promotors (Sacl-Fragment aus 15/G) in die Sacl-Schnittstelle des Konstrukts -2,45 ssll/GUS wurde das Konstrukt -4,68/GUS hergestellt, das den gesamten unter SEQ. ID No.1 beschriebenen Bereich (Nukleotide 1-4683) 5'-flankierend des ssll-Gens trägt.

Der 2443bp lange Bereich des Konstrukts -2,45 ssll/GUS wurde anschließend durch Deletionen weiter verkürzt. Die Deletionen wurden durch Restriktionen mit unterschiedlichen Restriktionsenzymen durchgeführt, wodurch Bereiche mit beschriebenen DNA-Elementen im Promotor entfernt wurden. Insgesamt wurden folgende Testkonstrukte des ssll -Promotors kloniert:
- 4,68 ssll / GUS
- 2,45 ssll / GUS (SacI-Restriktion an Position 2241, SEQ ID No.1);
- 2,05 ssll / GUS (KpnI-Restriktion an Position 2637, SEQ ID No.1);
- 1,11 ssll / GUS (SpeI-Restriktion an Position 3567, SEQ ID No.1);
- 0,61 ssll / GUS (HindIII-Restriktion an Position 4071, SEQ ID No.1);
- 0,53 ssll / GUS (SphI-Restriktion an Position 4151, SEQ ID No.1) und
- 0,28 ssll / GUS (NotI-Restriktion an Position 4403, SEQ ID No.1);

### 6. Transiente Expressionsanalysen der Promotor-Testvektoren

Die Funktionsfähigkeit der isolierten Promotorkonstrukte wurde in transienten Expressionsanalysen überprüft. Die Tests wurden mit den aus Beispiel 5 erhaltenen ssll-Promotortestvektoren und ihren Deletionskonstrukten durchgeführt.

Die transienten Expressionsanalysen erfolgten nach biolistischer Transformation von verschiedenen Geweben (Karyopsen, Embryonen, Blättern, Wurzeln) von Weizen. Die Transformation von Embryonen, Blättern und Wurzeln wurde nach Becker *et al*. (Plant J. (1994) 5 (2): 229-307) durchgeführt, während die biolistische Transformation des Endosperms von Karyopsen modifiziert nach Mena *et al*. (Plant J. (1998) 16(1), 53-62) erfolgte. Der Nachweis der Reportergenaktivität erfolgte durch histochemischen Nachweis der GUS-Aktivität (Jefferson (1987) Plant Molecular Biology Reporter Vol.5 (4): 387-405). Die Experimente an 10-30 Tage alten (dap), längs- und quergeschnittenen Weizenkaryopsen zeigten, daß der ssll-Promotor zu einer Expression des Reportergens im Stärke-Endosperm der Karyopse führt. In anderen Geweben (Perikarp, Blättern, Wurzeln) konnte keine GUS-Aktivität nachgewiesen werden.

Folgende Deletionskonstrukte des ssll-Promotors erwiesen sich in transienten Expressionsanalysen als funktionsfähig:
- 4,68 ssll / GUS
- 2,45 ssll / GUS (Sacl-Restriktion an Position 2241, SEQ ID No.1)
- 2,05 ssll / GUS (Kpnl-Restriktion an Position 2637, SEQ ID No.1);
- 1,11 ssll / GUS (Spel-Restriktion an Position 3567, SEQ ID No.1)
- 0,61 ssll / GUS (HindIII-Restriktion an Position 4071, SEQ ID No.1)

Das Konstrukt -0,28 SSII / GUS (Notl-Restriktion an Position 4.403; SEQ ID No.1) zeigte dagegen keine GUS-Reportergenaktivität mehr.

Die Ergebnisse transienter Expressionsanalysen eines Promotor-Reportergenkonstrukts entsprechen nicht immer dem Expressionsmuster nach stabiler Integration in das Pflanzengenom. Darüber hinaus können die Ergebnisse transienter Expressionsanalysen nach Mikroprojektilbeschuß aufgrund der Variabilität getroffener Zellen stark variieren. Es wurden daher weitere Analysen zur Gewebespezifität der isolierten Promotoren an stabil transformierten Pflanzen durchgeführt.

### 7. Stabile Transformation von Weizen mit den Promotor-Testvektoren

Zur Erzeugung stabil transformierter Weizenpflanzen wurden der in Beispiel 5 beschriebene Promotortestvektor -4,68 ssll/GUS und die dort ebenfalls beschriebenen Deletionskonstrukte -2,45 ssll/GUS und -0,61 ssll/GUS verwendet.

Die Herstellung der transgenen Pflanzen erfolgte nach der Methode von Becker *et al.* (Plant J. (1994) 5 (2): 229-307). Als Selektionsmarker wurde das phosphinothricin-Resistenz tragende Plasmid p35S-PAT (enthaltend das pat-Gen, Aventis CropScience GmbH, Frankfurt) eingesetzt.

### 8. Analyse der GUS-Reportergenexpression in stabil transformierten Weizenpflanzen

Die funktionale Analyse der ssll-Promotorfragmente erfolgte nach der Regeneration der transgenen Pflanzen und dem Nachweis einer stabilen und vollständigen Integration der Testkonstrukte in das Weizengenom über Southern-Analysen.

Die Reportergenaktivität in den regenerierten transgenen Pflanzen wurde über einen histochemischen GUS-Nachweis untersucht. Verschiedene Gewebe der transgenen Pflanzen (Blätter, Wurzeln, Endosperm, Embryo, Pollen) wurden analysiert. Eine Reportergenaktivität konnte ausschließlich in den Karyopsen der transgenen Pflanzen nachgewiesen werden. Hier zeigte sich die GUS-Aktivität im zentralen Stärkeendosperm lokalisiert. Im Embryo, dem Aleuron und der Embryo-umgebenden Region wurde dagegen keine GUS-Aktivität nachgewiesen. Auch im assimilierenden Gewebe der Blätter, sowie in den Wurzeln und im Pollen konnte keine Reportergen-Aktivität nachgewiesen werden.

Die unter SEQ ID No.1 beschriebenen 4683bp 5'-flankierend des ssll-Gens zeigen in den durchgeführten Funktionsanalysen eine gewebespezifische Expression des Transgens ausschließlich im Stärkeendosperm der Karyopsen. Auch die deletierten Fragmente des isolierten Promotors (Position 2241 bis 4683, bzw. 4071 bis 4683) vermitteln weiterhin eine gewebespezifische Expression des Transgens.

## Patentansprüche

1. Nukleinsäuremolekül mit der Funktion eines karyopsenspezifischen Promotors, das
a) die durch Nukleotid 1-4683 von Seq ID No. 1 definierte Nucleinsäuresequenz umfaßt;
b) ein oder mehrere Sequenzelemente umfaßt, ausgewählt aus der Gruppe bestehend aus Seq ID No. 2; Seq ID No. 3; Seq ID No. 4; Seq ID No. 5; Seq ID No. 6; Seq ID No. 7; Seq ID No. 8; Seq ID No. 9 und Seq ID No. 10;
c) einen funktionalen Teil der in a) genannten Nucleinsäuresequenz umfaßt; und/oder
d) eine Sequenz umfaßt, die mit einer der in a) genannten Nucleinsäuresequenzen zu etwa 60-99 %, vorzugsweise zu etwa 75-99 %, insbesondere zu etwa 90-99 % und ganz besonders bevorzugt zu etwa 95-99 % identisch ist.

2. Nukleinsäuremolekül nach Anspruch 1, der ein in monokotylen Pflanzen aktiver Promotor ist.

3. Expressionskassette enthaltend ein Nukleinsäuremolekül nach einem der Ansprüche 1-2.

4. Vektor enthaltend ein Nukleinsäuremolekül nach einem der Ansprüche 1-2 oder eine Expressionskassette nach Anspruch 3.

5. Vektor nach Anspruch 4, der zur Transformation von pflanzlichen Zellen geeignet ist.

6. Wirtszelle, die genetisch modifiziert ist mit einem Nukleinsäuremolekülen nach einem der Ansprüche 1-2, mit einer Expressionskassette nach Anspruch 3 oder einem Vektor nach einem der Ansprüche 4-5.

7. Wirtszelle nach Anspruch 6, die eine pro- oder eukaryontische Zelle ist.

8. Wirtszelle nach Anspruch 6, die eine Pflanzenzelle ist.

9. Pflanze enthaltend Pflanzenzellen nach Anspruch 8.

10. Vermehrungsmaterial oder Emtegut von Pflanzen nach Anspruch 9, enthaltend Pflanzenzellen nach Anspruch 8.

11. Verfahren zur Herstellung transgener Pflanzenzellen nach Anspruch 8, worin man Pflanzenzellen, -gewebe oder -teile oder Protoplasten mit einem Nukleinsäuremolekül nach einem der Ansprüche 1-2, mit einem Vektor nach einem der Ansprüche 4-5, mit einer Expressionskassette nach Anspruch 3 oder mit einer Wirtszelle nach Anspruch 6 transformiert, und die transformierten Pflanzenzellen, gewebe, -teile oder Protoplasten in einem Wachstumsmedium kultiviert.

12. Verfahren zur Herstellung transgener Pflanzen nach Anspruch 9, worin man Pflanzenzellen, -gewebe oder -teile oder Protoplasten mit einem Nukleinsäuremolekül nach einem der Ansprüche 1-2, mit einem Vektor nach einem der Ansprüche 4-5, mit einer Expressionskassette nach Anspruch 3 oder mit einer Wirtszelle nach Anspruch 6 transformiert, die transformierten Pflanzenzellen, -gewebe, -teile oder Protoplasten in einem Wachstumsmedium kultiviert und aus den erhaltenen Pflanzenzellen ganze Pflanzen regeneriert.

13. Verwendung eines Nukleinsäuremoteküls nach einem der Ansprüche 1-2 zur karyopsenspezifischen Expression von Genen in genetisch modifizierten Pflanzen.

14. Verwendung eines Nukleinsäuremoleküls nach einem der Ansprüche 1-2 zur karyopsenspezifischen Suppression von Genen in genetisch modifizierten Pflanzen.

15. Verfahren zur karyopsenspezifischen Genexpression in Pflanzen, worin ein Nukleinsäuremolekül gemäß einem der Ansprüche 1-2 stabil in das Genom einer Pflanzenzelle integriert wird und aus besagter Pflanzenzelle eine Pflanze regeneriert wird.

16. Verfahren zur karyopsenspezifischen Gensuppression in Pflanzen, worin ein Nukleinsäuremolekül gemäß einem der Ansprüche 1-2 stabil in das Genom einer Pflanzenzelle integriert wird und aus besagter Pflanzenzelle eine Pflanze regeneriert wird.

## Claims

1. A nucleic acid molecule with the function of a caryopsis-specific promoter, which nucleic acid molecule
a) comprises the nucleic acid sequence defined by nucleotide 1-4683 of Seq ID No. 1;
b) comprises one or more sequence elements selected from the group consisting of Seq ID No.2; Seq ID No. 3; Seq ID No. 4; Seq ID No.5; Seq ID No. 6; Seq ID No. 7; Seq ID No.8; Seq ID No.9 and Seq ID No.10;
c) comprises a functional portion of the nucleic acid sequence mentioned under a); and/or
d) comprises a sequence which has approx. 60-99% identity, preferably approx. 75-99% identity, in particular approx. 90-99% identity and very especially preferably approx. 95-99% identity with one of the nucleic acid sequences mentioned under a).

2. The nucleic acid molecule according to claim 1, which is a promoter active in monocotyledonous plants.

3. An expression cassette comprising a nucleic acid molecule according to claim 1 or 2.

4. A vector comprising a nucleic acid molecule according to claim 1 or 2 or an expression cassette according to claim 3.

5. The vector according to claim 4 which is suitable for transforming plant cells.

6. A host cell, which is genetically modified with a nucleic acid molecule according to claim 1 or 2, with an expression cassette according to claim 3 or with a vector according to claim 4 or 5.

7. The host cell according to claim 6, which is a pro- or eukaryotic cell.

8. The host cell according to claim 6, which is a plant cell.

9. A plant comprising plant cells according to claim 8.

10. Propagation material or harvested material from plants according to claim 9, comprising plant cells according to claim 8.

11. A method of preparing transgenic plant cells according to claim 8, wherein plant cells, plant tissue, plant parts or protoplasts are transformed with a nucleic acid molecule according to claim 1 or 2, with a vector according to claim 4 or 5, with an expression cassette according to claim 3 or with a host cell according to claim 6, and the transformed plant cells, plant tissues, plant parts or protoplasts are cultivated in a growth medium.

12. A method of preparing transgenic plants according to claim 9, wherein plant cells, plant tissue, plant parts or protoplasts are transformed with a nucleic acid molecule according to claim 1 or 2, with a vector according to claim 4 or 5, with an expression cassette according to claim 3 or with a host cell according to claim 6, the transformed plant cells, plant tissues, plant parts or protoplasts are grown in a growth medium, and intact plants are regenerated from the obtained plant cells.

13. Use of a nucleic acid molecule according to claim 1 or 2 for the caryopsis-specific expression of genes in genetically modified plants.

14. Use of a nucleic acid molecule according to claim 1 or 2 for the caryopsis-specific suppression of genes in genetically modified plants.

15. A method for the caryopsis-specific gene expression in plants, wherein a nucleic acid molecule according to claim 1 or 2 is stably integrated into the genome of a plant cell, and the plant is regenerated from said plant cell.

16. A method for the caryopsis-specific gene suppression in plants, wherein a nucleic acid molecule according to claim 1 or 2 is stably integrated into the genome of a plant cell, and a plant is regenerated from said plant cell.

## Revendications

1. Molécule d'acide nucléique qui assure la fonction d'un promoteur spécifique dans les caryopses,
a) qui comprend la séquence d'acide nucléique définie par les nucléotides 1 à 4683 de la SEQ ID No:1;
b) qui comprend un ou plusieurs éléments de séquence sélectionnés dans le groupe constitué de la SEQ ID No:2, de la SEQ ID No:3, de la SEQ ID No:4, de la SEQ ID No:5, de la SEQ ID No:6, de la SEQ ID No:7, de la SEQ ID No:8, de la SEQ ID No:9 et de la SEQ ID No:10,
c) qui comprend une partie fonctionnelle de la séquence d'acide nucléique mentionnée en a) et/ou
d) qui comporte une séquence qui est identique à environ 60-99%, de préférence à environ 75-99%, en particulier à environ 90-99% et de façon tour particulièrement préférable à environ 95-99% à la séquence d'acide nucléique mentionnée en a).

2. Molécule d'acide nucléique selon la revendication 1, qui est un promoteur actif dans les plantes monocotylées.

3. Cassette d'expression qui contient une molécule d'acide nucléique selon l'une des revendications 1 et 2.

4. Vecteur qui contient une molécule d'acide nucléique selon l'une des revendications 1 et 2 ou une cassette d'expression selon la revendication 3.

5. Vecteur selon la revendication 4 qui convient pour transformer des cellules végétales.

6. Cellule-hôte qui a été modifiée génétiquement par une molécule d'acide nucléique selon l'une des revendications 1 et 2, une cassette d'expression selon la revendication 3 ou un vecteur selon l'une des revendications 4 et 5.

7. Cellule-hôte selon la revendication 6, qui est une cellule procaryote ou eucaryote.

8. Cellule-hôte selon la revendication 6, qui est une cellule végétale.

9. Plante qui contient des cellules végétales selon la revendication 8.

10. Matériau de multiplication ou produit de plantes selon la revendication 9, qui contient des cellules végétales selon la revendication 8.

11. Procédé de création de cellules transgéniques de plantes selon la revendication 8, dans lequel on transforme des cellules, des tissus ou des parties de plantes ou des protoplastes avec une molécule d'acide nucléique selon l'une des revendications 1 et 2, un vecteur selon l'une des revendications 4 et 5, une cassette d'expression selon la revendication 3 ou une cellule-hôte selon la revendication 6, et dans lequel on cultive dans un milieu de croissance les cellules, tissus, parties ou protoplastes de plantes.

12. Procédé de création de plantes transgéniques selon la revendication 9, dans lequel on transforme des cellules, des tissus ou des parties de plantes ou des protoplastes avec une molécule d'acide nucléique selon l'une des revendications 1 et 2, avec un vecteur selon l'une des revendications 4 et 5, une cassette d'expression selon la revendication 3 ou une cellule-hôte selon la revendication 6, et dans lequel on cultive dans un milieu de croissance les cellules, tissus, parties ou protoplastes de plantes transformées et dans lequel on régénère des plantes complètes à partir des cellules de plantes ainsi obtenues.

13. Utilisation d'une molécule d'acide nucléique selon l'une des revendications 1 à 2 pour l'expression, spécifique dans les caryopses, de gènes dans des plantes génétiquement modifiées.

14. Utilisation d'une molécule d'acide nucléique selon l'une des revendications 1 et 2 pour l'inhibition, spécifique dans les caryopses, de gènes dans des plantes génétiquement modifiées.

15. Procédé d'expression, spécifique dans les caryopses, de gènes de plantes, dans lequel une molécule d'acide nucléique selon l'une des revendications 1 et 2 est intégrée de manière stable dans le génome d'une cellule de plante et une plante est régénérée à partir de ladite cellule de plante.

16. Procédé pour l'inhibition, spécifique dans les caryopses, de gènes de plantes, dans lequel une molécule d'acide nucléique selon l'une des revendications 1 et 2 est intégrée de manière stable dans le génome d'une cellule de plante et une plante est régénérée à partir de ladite cellule de plante.
